# EUROPEAN PATENT SPECIFICATION

(11) **EP 3 199 619 B1**
(45) Date of publication and mention of the grant of the patent: **01.07.2026**
(21) Application number: 15841770.9
(22) Date of filing: 14.09.2015
(51) Int. Cl.: C12N 1/00, C12M 1/00, C12M 1/06, C12N 1/20, C12M 1/26, C12M 1/02, C12M 1/34, C12P 7/06

(54) **MICROORGANISM CULTURE METHOD AND CULTURE APPARATUS**
KULTURVERFAHREN FÜR MIKROORGANISMEN UND KULTURAPPARAT
PROCÉDÉ DE CULTURE DE MICRO-ORGANISMES ET APPAREIL DE CULTURE

(30) Priority: 19.09.2014 JP 2014191858; 19.09.2014 JP 2014191859
(43) Date of publication of application: 02.08.2017
(73) Proprietor: Sekisui Chemical Co., Ltd., Osaka-shi, Osaka 530-8565 (JP)
(72) Inventor: ISHII Tetsuya, Tsukuba-shi Ibaraki 300-4292 (JP); SATOU Kanetomo, Tsukuba-shi Ibaraki 300-4292 (JP); FUJIMORI Yoji, Tsukuba-shi Ibaraki 300-4292 (JP); HAMACHI Kokoro, Tsukuba-shi Ibaraki 300-4292 (JP); NISHIYAMA Norihide, Tsukuba-shi Ibaraki 300-4292 (JP)
(74) Representative: Ter Meer Steinmeister & Partner
(86) International application number: PCT/JP2015/076043
(87) International publication number: WO 2016/043163

(56) References cited:
- WO-A1-2009/114127
- WO-A1-2013/119119
- DE-A1- 102006 035 213
- DE-B4- 102006 035 213
- JP-A- 2007 082 437
- JP-A- 2007 082 438
- JP-A- 2011 512 870
- JP-A- 2014 050 406
- US-A- 5 079 161
- US-A1- 2012 288 928
- US-A1- 2013 065 282
- US-A1- 2013 330 792

## Description

### Field of the Invention

The present invention relates to a method and an apparatus for culturing microorganisms and particularly relates to a culture method and a culture apparatus for culturing gas-utilizing microorganisms that produce valuable materials from a substrate gas by fermentation.

### Background of the Invention

According to Patent Document 1, gas-utilizing microorganisms are cultured in a culture solution in a culture tank, providing a substrate gas containing CO₂ and H₂ to the culture solution. Valuable materials such as acetate are produced by fermentation activities of the gas-utilizing microorganisms. To extract the valuable materials, a portion of the culture solution is taken out from the culture tank and separated into a concentrated culture solution and a microorganisms-removed solution by a separator. The concentrated culture solution is returned to the culture tank and the microorganisms-removed solution is discharged to a next step.

### Prior Art Documents

### Patent Documents

Patent Document 1: United States Patent Application Publication No. US2013/0065282
US2013/330792 A1 relates to a method of producing a chemical by continuous fermentation that includes a fermentation step of converting a fermentation feedstock to a fermentation liquid containing a chemical by fermentation on cultivation of a microorganism; a membrane separation step of recovering the chemical as a filtrate by a separation membrane from the fermentation liquid; a concentrating step of obtaining a permeate and a concentrate containing the chemical by a reverse osmosis membrane from the filtrate; and/or a purification step of distilling the filtrate to increase a purity of the chemical, in which, cleaning of the separation membrane in the membrane separation step is performed by using the permeated liquid from the reverse osmosis membrane in the concentrating step and/or the condensed liquid in the purification step.
WO2009/114127 A1 relates to a method for sustaining microorganism culture in a syngas fermentation reactor in decreased concentration or absence of various substrates comprising: adding carbon dioxide and optionally alcohol; maintaining free acetic acid concentrations; and performing the above mentioned steps within specified time.

### Summary of the Invention

### Problem to be Solved by the Invention

When the inventors tried to culture gas-utilizing microorganisms of this kind, they encountered a problem that generally all of the individuals of the microorganisms are uniformly weakened and easily die when their living environment in a culture tank is deteriorated due to a decrease in supplied amount for some reason or stoppage of supply of a substrate gas or some constituents of the substrate gas.

The culture apparatus of the Patent Document 1 may be effective when microorganisms stay in a culture tank longer than a culture solution. However, when it is necessary to discharge a portion of the microorganisms from the culture tank for the sake of stable culturing according to a degree of increase in a population of the microorganisms or a supply situation of the substrate gas, the portion of the microorganisms may have to be discharged together with the culture solution containing them. Therefore, the microorganisms cannot be discharged outside of the system faster than the culture solution. In other words, a discharge speed of the microorganisms cannot be faster than a discharge speed of the culture solution. To increase the discharge speed of the microorganisms, it is necessary to increase the discharge speed of the culture solution. This may result in an increase in a discharge amount, which is not preferable.

**In** view of the above, it is a first object of the present invention to culture gas-utilizing microorganisms in a stable manner regardless of variations in supply situation of a substrate gas.

**In** addition to the stable culturing of the microorganisms in a culture tank, it is a second object of the present invention to prevent waste of the culture solution when it is necessary to discharge a portion of the microorganisms from the culture tank.

### Means for Solving the Problems

To achieve the first object mentioned above, a method of the present invention provides a culture method for culturing gas-utilizing microorganisms that produce valuable materials from a substrate gas by fermentation, the method including steps as defined in claim 1.

According to this method, when a supply situation of the substrate gas or the predetermined constituent thereof is deteriorated, a population of the gas-utilizing microorganisms in the culture tank can be reduced by rapidly discharging the culture solution and thus the gas-utilizing microorganisms from the culture tank. By this arrangement, an amount of the substrate gas that each of the gas-utilizing microorganisms intakes can be surely secured. As a result, death of an entire population of the gas-utilizing microorganisms in the culture tank can be avoided, and the gas-utilizing microorganisms can be stably cultured. After discharging certain amount of the culture solution from the culture tank, it is preferable to return the discharge amount to a vicinity of the discharge amount before the discharge amount was increased. When the supply situation of the substrate gas or the predetermined constituent thereof is recovered, the fermentation by the gas-utilizing microorganisms can be rapidly restored and growth of the gas-utilizing microorganisms may progress.

"A supply flow rate becomes equal to or lower than a predetermined value" mentioned above means that the supply flow rate becomes approximately equals to or below 30% to 80%, preferably approximately equals to or below 50% of the supply flow rate in the normal operation mode. Alternatively, it may mean that in a case where there are n number ("n" is an integer equal to or greater than 2) of substrate gas supply apparatus, the supply flow rate becomes (n-m)/n times the supply flow rate in the normal operation mode (when the supply flow rates are the same among the substrate gas supply apparatus in the normal operation mode), for example, by operation of m number ("m" is an integer equal to or greater than 1 and equal to or smaller than "n") of substrate gas supply apparatus being suspended due to troubles or maintenance or the like.

The "supply situation of the substrate gas or the predetermined constituent thereof" includes a flow rate of the substrate gas, a flow rate of the predetermined constituent of the substrate gas, a partial pressure of the predetermined constituent, a pressure of the substrate gas and a temperature of the substrate gas or the like. Preferably, the "predetermined constituent" is constituent of the substrate gas that is particularly effective for live activities of the gas-utilizing microorganisms, including survival, fermentation and growth. The "controlling a discharge amount" includes not only increasing or decreasing the amount of discharge in a state in which discharging is carried out but also starting discharging in a state in which discharging is stopped, stopping discharging in a state in which discharging is carried out and maintaining the amount of discharge in a state in which discharging is carried out. "Increase the discharge amount" as used in this specification includes not only increasing the amount of discharging in a state in which discharging is carried out but also starting discharging in a state in which discharging is stopped. "The discharge amount" may be a discharge amount per unit time (discharge flow rate) or may be a total discharge amount in a single discharge operation.

It is preferable to temporarily increase the discharge amount in the step of rapidly discharging. "To temporarily increase the discharge amount" includes to make a discharge amount per unit time (discharge flow rate) during a certain period greater than before and to decrease the discharge amount per unit time (discharge flow rate) after the passage of the certain period. It also includes to make a discharge amount at one or a plurality of discharge operations greater than before and after the one or the plurality of discharge operations in a case where the discharge operations are carried out intermittently or periodically by batch processing. After the rapidly discharging starts, the increased discharge amount may be maintained until an end of the culturing.

Preferably, the culture solution is replenished to the culture tank according to an amount of the culture solution rapidly discharged from the culture tank. Preferably, the gas-utilizing microorganisms are not or hardly contained in the culture solution to be replenished. This allows for maintaining an amount of the culture solution in the culture tank at generally the same before and after the rapidly discharging. Moreover, a concentration of the gas-utilizing microorganisms in the culture solution in the culture tank can be lowered than the concentration thereof before the rapidly discharging. Therefore, an amount of the substrate gas that each of the gas-utilizing microorganisms intakes can be surely secured, and the death of the entire population of the gas-utilizing microorganisms can be sufficiently avoided.

Preferably, the present method further includes steps of:
separating the discharged culture solution into a concentrated culture solution and a diluted culture solution, the gas-utilizing microorganisms being concentrated in the concentrated culture solution, the gas-utilizing microorganisms being diluted in the diluted culture solution;
returning the diluted culture solution to the culture tank; and
sending out the concentrated culture solution to subsequent apparatus including an extraction part that extracts the valuable materials or a storage tank for extraction or a discharge solution treatment part.

"Diluted" mentioned above includes a state in which the gas-utilizing microorganisms in the discharged culture solution are completely removed. "Diluted culture solution" includes a culture solution whose concentration of the gas-utilizing microorganisms is zero.

Preferably, in the step of separating, the discharged culture solution is circulated along a circulation passage in which, of a filter and a storage tank for concentration, at least the filter is disposed.

By this arrangement, even when the filter is a cross-flow filter, for example, the discharged culture solution can be effectively separated into the diluted culture solution and the concentrated culture solution, and a highly-concentrated culture solution can be obtained.

Preferably, the present method further includes steps of:
monitoring a concentration of the gas-utilizing microorganisms in the culture solution in the culture tank or in the discharged culture solution; and
controlling a separation ratio between the concentrated culture solution and the diluted culture solution so that the concentration may be a predetermined concentration.

Preferably, the present method further includes steps of:
separating the discharged culture solution into a concentrated culture solution and a diluted culture solution, the gas-utilizing microorganisms being concentrated in the concentrated culture solution, the gas-utilizing microorganisms being diluted in the diluted culture solution;
storing the diluted culture solution; and
returning the stored diluted culture solution to the culture tank at the same time as or slightly before or after the rapid discharge of the culture solution.

By this arrangement, the stored diluted culture solution can be utilized as a replenishment culture solution to the culture tank in the step of rapidly discharging. Replenishment allows for securing the amount of the culture solution in the culture tank and maintaining composition of the culture solution generally the same as before the step of rapidly discharging. Therefore, the gas-utilizing microorganisms can be prevented from being damaged by change in composition of the culture solution.

Preferably, the diluted culture solution obtained in the step of separating is returned to the culture tank (preferably without being stored) after the rapid discharge until the supply flow rate is recovered to the predetermined value or higher; and the concentrated culture solution obtained in the step of separating is sent out to the subsequent apparatus including the extraction part that extracts the valuable materials or the storage tank for extraction or the discharge solution treatment part (preferably without being returned to the culture tank).

By this arrangement, more gas-utilizing microorganisms in the culture solution than liquid constituents thereof can be discharged from the culture tank. Accordingly, even if the gas-utilizing microorganisms grow in the culture tank, the concentration of the gas-utilizing microorganisms in the culture solution can be maintained at a concentration suitable for a gas supply flow rate. Therefore, the death of the entire population of the gas-utilizing microorganisms due to insufficient supply flow rate can be sufficiently avoided.

Preferably, the concentrated culture solution obtained in the step of separating is returned to the culture tank (preferably without being sent out to the subsequent apparatus) when the supply flow rate becomes recoverable to the predetermined value or higher.

By this arrangement, the concentration of the gas-utilizing microorganisms in the culture tank can be rapidly increased to be returned to the concentration before the supply flow rate became the predetermined value and lower. At this time, it is preferable that the diluted culture solution obtained in the step of separating is stored.

Preferably, the present method further includes a step of making a temperature of the diluted culture solution under storage higher or lower than a temperature of the culture tank.

By this arrangement, breeding of bacteria in the diluted culture solution that is being stored can be suppressed or prevented.

Preferably, the present method further includes a step of exchanging heat between the diluted culture solution to be returned to the culture tank and the rapidly discharged culture solution.

By this arrangement, the diluted culture solution can be replenished to the culture tank with the temperature of the diluted culture solution being brought near to the temperature of the culture tank. Therefore, the gas-utilizing microorganisms in the culture tank can be prevented or suppressed from being damaged by change of liquid temperature. Moreover, thermal efficiency can be enhanced by utilizing the discharged culture solution as a heat source.

Preferably, the present method further includes a step of backwashing the filter for the step of separating by at least a portion of the diluted culture solution to be returned to the culture tank.

By this arrangement, the filter can be efficiently constrained from being clogged.

To achieve the first object mentioned above, an apparatus of the present invention provides a culture apparatus for culturing gas-utilizing microorganisms that produce valuable materials from a substrate gas by fermentation, wherein the apparatus is as defined in claim 12.

**In** this apparatus, when the supply situation of the substrate gas is deteriorated, an amount of the substrate gas that each of the gas-utilizing microorganisms in the culture tank intakes can be secured by rapidly discharging the culture solution and thus the gas-utilizing microorganisms from the culture tank. Therefore, death of the entire population of the gas-utilizing microorganisms in the culture tank can be avoided, and the gas-utilizing microorganisms can be stably cultured. When the supply flow rate of the substrate gas is restored, fermentation by the gas-utilizing microorganisms is rapidly restored and the growth is promoted.

Preferably, the apparatus further includes a rapid replenishment passage, the culture solution being replenished to the culture tank according to an amount of the culture solution rapidly discharged through the rapid replenishment passage. Preferably, the gas-utilizing microorganisms are not or hardly contained in the culture solution to be replenished. A culture medium may be used as a replenishment culture solution. The diluted culture solution obtained from the discharged culture solution may be used as the replenishment culture solution.

Preferably, the apparatus of the present invention further includes:
a separation part that separates the discharged culture solution into a concentrated culture solution and a diluted culture solution, the gas-utilizing microorganisms being concentrated in the concentrated culture solution, the gas-utilizing microorganisms being diluted in the diluted culture solution;
a diluted solution return passage that returns the diluted culture solution to the culture tank; and
a concentrated solution send-out passage that sends out the concentrated culture solution to subsequent apparatus including an extraction part that extracts the valuable materials or a storage tank for extraction or a discharge solution treatment part.

Preferably, the separation part includes: a circulation passage, the concentrated culture solution being circulated in the circulation passage; and a filter disposed in the circulation passage.

Preferably, a storage tank for concentration is disposed in the circulation passage.

Preferably, the apparatus of the present invention further includes:
a microbial concentration measuring instrument that measures a concentration of the gas-utilizing microorganisms in the culture solution in the culture tank; and
a separation ratio control part that controls a separation ratio between the concentrated culture solution and the diluted culture solution in the separation part so that the concentration may be a predetermined concentration.

Preferably, the apparatus of the present invention further includes:
a separation part that separates the discharged culture solution into a concentrated culture solution and a diluted culture solution, the gas-utilizing microorganisms being concentrated in the concentrated culture solution, the gas-utilizing microorganisms being diluted in the diluted culture solution; and
a diluted solution storage tank that stores the diluted culture solution, wherein the rapid replenishment passage extends from the diluted solution storage tank to the culture tank.

Preferably, the diluted solution storage tank is provided with a liquid temperature conditioner that makes a temperature of the diluted culture solution higher or lower than a temperature of the culture tank.

Preferably, the apparatus of the present invention further includes a heat exchanger that exchanges heat between the diluted culture solution in the rapid replenishment passage and the rapidly discharged culture solution.

Preferably, the valuable materials are extracted from the discharged culture solution.

Thereby, the valuable materials can be obtained and provided for various uses.

More preferably, the discharged culture solution is stored in a storage tank as a stored solution, and the valuable materials are extracted from the stored solution.

By this arrangement, the discharged culture solution can be stored as the stored solution, and then extracted as appropriate according to an ability of an extraction part to extract valuable materials and a demand for the valuable materials or the like. Even when a discharge flow rate is great, leakage of the culture solution to outside can be avoided and overloading of the extraction part can be avoided.

Preferably, the concentration of the gas-utilizing microorganisms in the discharged culture solution is higher than the concentration of the gas-utilizing microorganisms in the culture solution other than the part of the culture solution in the culture tank.

By this arrangement, waste of liquid constituents of the culture solution can be curtailed. Moreover, in a case where the culture medium is replenished to the culture tank in an amount corresponding to the discharged amount, the amount of replenishment can be reduced. Therefore, change in composition of the culture solution in the culture tank can be reduced, and a probability of the gas-utilizing microorganisms dying of shock can be reduced.

Preferably, the storage tank is connected to the discharge part and the extraction part that extracts the valuable materials is connected to the storage tank.

By this arrangement, the culture solution discharged from the culture tank can be stored in the storage tank, and then extracted as appropriate according to the ability of the extraction part to extract valuable materials and a demand for the valuable materials or the like. Even when the discharge flow rate of the culture solution is great, leakage of the culture solution to outside can be avoided and overloading of the extraction part can be avoided.

To achieve the second object mentioned above, a method of the present invention provides a culture method for culturing microorganisms that produce valuable materials by fermentation including:
culturing the microorganisms in a culture solution in a culture tank;
obtaining concentrated culture solution by concentrating the microorganisms contained in a portion of the culture solution; and
feeding the concentrated culture solution to subsequent steps including a step of extracting the valuable materials and a step of treating discharge solution.

According to this method, the microorganisms in the culture tank can be stably cultured by feeding a part of the microorganisms from the culture tank to the subsequent steps as the concentrated culture solution constantly or as appropriate according to a culture state including a degree of increase in the population of the microorganisms and a supply situation of a substrate gas. Moreover, as much liquid constituents as possible can be retained in a system by concentrating the microorganisms before bringing them outside of the system. **In** other words, a discharge speed of the microorganisms can be faster than a discharge speed of the culture solution. This allows for prevention of waste of the culture solution. The valuable materials can be extracted from the concentrated culture solution and discharge solution can be treated in the subsequent steps.

Preferably, the portion of the culture solution is discharged from the culture tank;
the discharged culture solution is separated into the concentrated culture solution and a diluted culture solution in which the microorganisms are diluted;
the diluted cultured solution is returned to the culture tank; and
the concentrated culture solution is sent out to subsequent apparatus including an extraction part that extracts the valuable materials or a storage tank for extraction or a discharge solution treatment part.

By this arrangement, the microorganisms contained in the portion of the culture solution can be surely concentrated and the concentrated culture solution can be surely obtained. Moreover, returning the diluted culture solution to the culture tank allows for the prevention of the waste of the culture solution, which allows for reduction of cost. Moreover, maintaining an environment inside the culture tank (composition of the culture solution or the like) as constant as possible allows for more stable culturing of the microorganisms.

Preferably, the concentration of the microorganisms in the culture solution or the portion of the culture solution in the culture tank is monitored; and
a separation ratio between the concentrated culture solution and the diluted culture solution is adjusted so that the concentration may be a predetermined concentration.

By this arrangement, the concentration of the microorganisms in the culture tank can be maintained at the predetermined concentration even when the microorganisms in the culture tank excessively grows by returning more diluted culture solution to the culture tank to reduce the microbial concentration in the culture tank. This allows for further stable culturing of the microorganisms.

Preferably, the portion of the culture solution from the culture tank is stored in a storage tank for concentration;
a stored solution in the storage tank for concentration is taken out and separated into a highly-concentrated culture solution in which the microorganisms are more highly-concentrated than in the stored solution and a diluted culture solution in which the microorganisms are diluted;
the diluted culture solution is returned to the culture tank;
the highly-concentrated culture solution is returned to the storage tank for concentration; and
a portion of the stored solution in the storage tank for concentration is fed to the subsequent steps.

The stored solution is a moderately-concentrated culture solution. Specifically, the stored solution is a mixture of the portion of the culture solution and the highly-concentrated culture solution. Therefore, the microbial concentration is higher in the stored solution than in the culture solution in the culture tank. In this method, a flow rate circulating between the storage tank for concentration and a separation film can be high, and clogging of the separation film can be prevented. Alternatively, the highly-concentrated culture solution may be fed to the subsequent steps.

Preferably, the microorganisms are gas-utilizing microorganisms that produce the valuable materials from a substrate gas by fermentation;
the substrate gas is supplied to the culture tank and dissolved in the culture solution; and
when a supply flow rate of the substrate gas to the culture tank becomes a predetermined flow rate or lower, the concentrated culture solution is produced and fed to the subsequent steps.

By this arrangement, when the supply flow rate of the substrate gas decreases, an amount of the substrate gas that each of the microorganisms intakes can be secured by reducing the population of the microorganisms in the culture tank, thereby death of the entire population of the microorganisms can be avoided.

To achieve the second object mentioned above, an apparatus of the present invention provides a culture apparatus for culturing microorganisms that produce valuable materials by fermentation, wherein the culture apparatus is as defined in claim 12 including:
a culture tank for receiving a culture solution for culturing the microorganisms therein;
a biomass concentration part for obtaining a concentrated culture solution by concentrating the microorganisms contained in a portion of the culture solution; and
a concentrated solution send-out passage that sends out the concentrated culture solution to subsequent apparatus including an extracting part that extracts the valuable materials or a storage tank for extraction or a discharge solution treatment part.

By using this apparatus, the microorganisms in the culture tank can be stably cultured by sending out a part of the microorganisms from the culture tank to the subsequent apparatus constantly or according to a culture state. Moreover, as much liquid constituents as possible can be retained in the culture apparatus by sending out the microorganisms to the subsequent apparatus after concentrating them. In other words, a discharge speed of the microorganisms can be faster than a discharge speed of the culture solution. Thereby, waste of the culture solution can be prevented. Moreover, the valuable materials can be extracted from the concentrated culture solution and discharge solution can be treated in the subsequent apparatus.

Preferably, the biomass concentration part includes a separation film that separates the portion of the culture solution from the culture tank into the concentrated culture solution and a diluted culture solution in which the microorganisms are diluted. Preferably, the culture apparatus further includes a return passage to tank for returning the diluted cultured solution to the culture tank.

By this arrangement, the microorganisms can be surely concentrated in the biomass concentration part and the waste of the culture solution can be surely prevented. Moreover, the microorganisms can be further stably cultured in the culture tank.

Preferably, the culture apparatus further includes a microbial concentration measuring instrument for measuring a concentration of the microorganisms in the culture solution in the culture tank; and
a separation ratio control part for controlling a separation ratio between the concentrated culture solution and the diluted culture solution in the biomass concentration part so that the concentration may be a predetermined concentration.

By this arrangement, excessive growth of the microorganisms in the culture tank can be suppressed and the concentration of the microorganisms can be maintained at the predetermined concentration. Therefore, the microorganisms can be further stably cultured.

Preferably, the biomass concentration part includes a storage tank for concentration in which the portion of the culture solution from the culture tank is stored; and
a separation film for separating a stored solution taken out from the storage tank for concentration into a highly-concentrated culture solution in which the microorganisms are more highly concentrated than in the stored solution and a diluted culture solution in which the microorganisms are diluted; and
the concentrated solution send-out passage extends from the storage tank for concentration.

Preferably, the culture apparatus includes a diluted solution return passage for returning the diluted culture solution to the culture tank and a return passage for re-concentration for returning the highly-concentrated culture solution to the storage tank for concentration.

The stored solution in the storage tank for concentration is a moderately-concentrated culture solution. Specifically, the stored solution in the storage tank for concentration is a mixture of the portion of the culture solution and the highly-concentrated culture solution. Therefore, the microbial concentration is higher in the stored solution than in the culture solution in the culture tank. In this aspect of the apparatus, a flow rate circulating between the storage tank for concentration and the separation film can be high, and the separation film can be constrained or prevented from being clogged.

Preferably, the microorganisms are gas-utilizing microorganisms that produce the valuable materials from a substrate gas by fermentation;
a supply passage for the substrate gas is connected to the culture tank; and
when a supply flow rate of the substrate gas to the culture tank becomes a predetermined flow rate or lower, the concentrated culture solution is produced in the biomass concentration part and sent out to the subsequent apparatus.

By this arrangement, when the supply flow rate of the substrate gas is decreased, an amount of the substrate gas that each of the microorganisms intakes can be secured by reducing the population of the microorganisms in the culture tank, thereby death of the entire population of the microorganisms can be avoided.

### Advantageous Effects of the Invention

According to the present invention, when the supply situation of the substrate gas is deteriorated, the population of the gas-utilizing microorganisms in the culture tank can be reduced by increasing the discharge amount of the gas-utilizing microorganisms from the culture tank. Thereby, the gas-utilizing microorganisms in the culture tank can be stably cultured regardless of variations in the supply situation of the substrate gas so as to avoid death of the entire population of the microorganisms.

### Brief Description of the Drawings

FIG. 1 is a schematic diagram showing a general configuration of a valuable materials generating system including a culture apparatus according to a first embodiment of the present invention.
FIG. 2 is a schematic diagram showing a general configuration of a valuable materials generating system including a culture apparatus according to a second embodiment of the present invention.
FIG. 3 is a schematic diagram showing a general configuration of a valuable materials generating system including a culture apparatus according to a third embodiment of the present invention.
FIG. 4 is a schematic diagram showing a valuable materials generating system including a culture apparatus according to a fourth embodiment of the present invention in a normal operation mode.
FIG. 5 is a schematic diagram showing the valuable materials generating system including the culture apparatus according to the fourth embodiment of the present invention in a rapidly-diluting operation mode.
FIG. 6 is a schematic diagram showing the valuable materials generating system including the culture apparatus according to the fourth embodiment of the present invention in a low biomass operation mode.
FIG. 7 is a schematic diagram showing the valuable materials generating system including the culture apparatus according to the fourth embodiment of the present invention in a state recovery operation mode.
FIG. 8 (a) is a schematic diagram showing a part of a valuable materials generating system including a culture apparatus according to a fifth embodiment of the present invention in a normal operation mode.
FIG. 8 (b) is a schematic diagram showing a part of the valuable materials generating system including the culture apparatus according to the fifth embodiment of the present invention in a rapidly diluting operation mode.
FIG. 9 is a schematic diagram showing a valuable materials generating system including a culture apparatus according to a sixth embodiment of the present invention in a rapidly-diluting operation mode.
FIG. 10 is a schematic diagram showing a general configuration of a valuable materials generating system including a culture apparatus according to a seventh embodiment of the present invention.
FIG. 11 is a schematic diagram showing a general configuration of a valuable materials generating system including a culture apparatus according to an eighth embodiment of the present invention.

### Mode for Carrying out the Invention

Embodiments of the present invention will be described hereinafter with reference to the drawings.

### <First Embodiment>

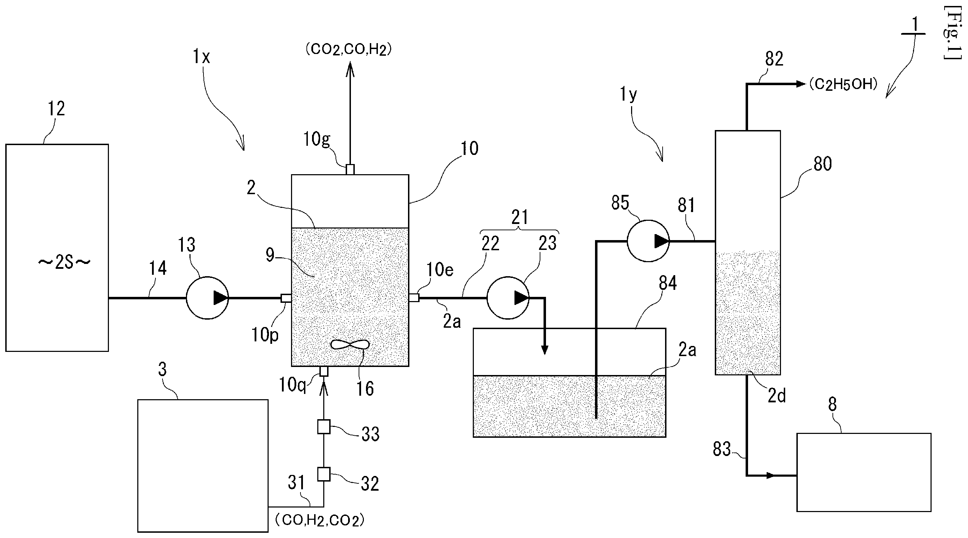

FIG. 1 shows a valuable materials generating system 1 according to a first embodiment of the present invention. The valuable materials generating system 1 includes a culture apparatus 1x and subsequent apparatus 1y. The culture apparatus 1x includes a culture tank 10 and a culture medium source 12. The subsequent apparatus 1y includes a storage tank 84 for extraction (breeding tank), a distillation tower 80 (extraction part) and a discharge solution treatment part 8.

A culture solution 2 is stored in the culture tank 10. Gas-utilizing microorganisms 9 are cultured in the culture solution 2. Anaerobic bacteria may be used as the gas-utilizing microorganisms 9 as disclosed in the Patent Document 1 (United States Patent Application Publication No. US2013/0065282), Japanese Patent Application Publication No. 2014-050406 and Japanese Patent Application Publication No. 2004-504058, for example. Valuable materials such as ethanol (C₂H₅OH) are synthesized from a substrate gas by fermentation activities of the gas-utilizing microorganisms 9. Substrate gas constituents (predetermined constituent) used for the fermentation of the gas-utilizing microorganisms 9 may be chiefly carbon monoxide (CO) and hydrogen (H₂). The valuable materials may include ethanol, butanol, ascetic acid or acetate and other organic compounds.

The culture solution 2 in the culture tank 10 is stirred by a stirrer 16. Therefore, the gas-utilizing microorganisms 9 are evenly dispersed throughout the culture solution 2.

The culture medium source 12 is connected to the culture tank 10. A culture medium 2S of the culture solution 2 is stored in the culture medium source 12. In other words, the culture solution 2 before the gas-utilizing microorganisms 9 are put therein is stored. The culture medium 2S is composed mostly of water (H₂O) with nutrient contents such as vitamins and phosphoric acids dispersed or dissolved therein. A culture medium supply passage 14 extends from the culture medium source 12. The culture medium supply passage 14 continues to a culture medium supply port 10p of the culture tank 10. A liquid sending pump 13 is disposed in an intermediate portion of the culture medium supply passage 14.

Moreover, a substrate gas source 3 is connected to the culture tank 10. Though not shown in detail in the drawings, the substrate gas source 3 may be an industrial waste disposal facility that treats industrial wastes or the like. In other words, the culture apparatus 1x, and thus the valuable materials generating system 1 is incorporated in an industrial waste disposal system. The substrate gas source 3 has a melting furnace. Wastes are burned in the melting furnace by a highly-concentrated oxygen gas and resolved to a low molecular level. Finally, an anaerobic substrate gas including carbon monoxide (CO), hydrogen (H₂) and carbon dioxide (CO₂) or the like is produced. A produced flow rate and composition of the substrate gas are not stable, depending on a kind and amount or the like of wastes.

A gas supply passage 31 extends from the substrate gas source 3. The gas supply passage 31 continues to a gas supply port 10q in a bottom portion of the culture tank 10. A gas flow meter 32 and a gas sensor 33 are disposed in intermediate portions of the gas supply passage 31. The gas flow meter 32 measures a flow rate of a gas passing through the gas supply passage 31. The gas sensor 33 may be a gas chromatography or the like and measures a composition (constituents and partial pressure or the like) of the gas passing through the gas supply passage 31.

A discharge port 10e (discharge part) is disposed in an intermediate portion or the bottom portion, for example, of the culture tank 10. A discharge passage 22 extends from the discharge port 10e to the subsequent apparatus 1y. The storage tank 84 for extraction is disposed at a downstream end of the discharge passage 22. The culture tank 10 and the storage tank 84 for extraction are connected via the discharge passage 22. A liquid sending pump 23 is disposed in an intermediate portion of the discharge passage 22. Preferably, the liquid sending pump 23 may be an inverter pump whose output can be controlled. The liquid sending pump 23 of the discharge passage 22 or the like constitutes a discharge control part 21. A flow rate control valve (not shown) may be further disposed in the discharge passage 22. The discharge control part 21 may include a flow rate control valve. The discharge control part 21 may include a controller (controlling member) that controls the liquid sending pump 23 and/or the flow rate control valve.

A discharged culture solution 2a from the culture tank 10 is stored in the storage tank 84 for extraction.

A send-out passage 81 is drawn from the storage tank 84 for extraction. A liquid sending pump 85 is disposed in the send-out passage 81. The liquid sending pump 85 prevents backflow. The send-out passage 81 continues to a middle portion of the distillation tower 80. An extracted liquid passage 82 extends from an upper end portion of the distillation tower 80. A discharge passage 83 extends from a bottom portion of the distillation tower 80. The discharge passage 83 continues to the discharge solution treatment part 8. Though not shown in detail in the drawings, the discharge solution treatment part 8 includes an anaerobic treatment part and an aerobic treatment part for a discharge solution or the like.

A culture method and a method for generating valuable materials by the valuable materials generating system 1 will be described hereinafter.

### <Culturing Step>

The culture medium 2S is introduced from the culture medium source 12 to the culture tank 10 and the gas-utilizing microorganisms 9 are cultured in the culture solution 2 in the culture tank 10. The gas-utilizing microorganisms 9 can be evenly dispersed throughout the culture solution 2 by stirring the culture solution 2 with the stirrer 16.

### <Substrate Gas Supplying Step>

The substrate gas (CO, CO₂, H₂, or the like) produced from the wastes in the substrate gas source 3 is introduced to the culture tank 10 via the gas supply passage 31 to dissolve the substrate gas in the culture solution 2 in the culture tank 10. The stirring may promote the dissolving of the substrate gas in the culture solution 2.

### <Fermentation Step>

**In** this step, the gas-utilizing microorganisms 9 in the culture solution 2 ferment to produce valuable materials such as ethanol from the substrate gas. Gas constituents such as CO₂ are also produced by the fermentation. Gas constituents such as CO₂ introduced via the gas supply passage 31, CO₂ produced by the fermentation and unused CO and H₂ or the like are discharged from the discharge port 10e of the culture tank 10. These gas constituents may be returned to the substrate gas source 3 or may be burned to be utilized as a heat source for distillation or the like.

### <Discharging Step>

### (1) Normal Operation Mode

The liquid sending pumps 13, 23 are constantly operated so that amounts of liquid sent by the liquid sending pumps 13, 23 may be balanced. Thereby, the culture medium 2S is sent from the culture medium source 12 to the culture tank 10 via the culture medium supply passage 14. A portion of the culture solution 2 in the culture tank 10 is discharged as the discharged culture solution 2a to the discharge passage 22 from the discharge port 10e. In the normal operation, the amount of liquid sent by the liquid sending pump 13 (supply flow rate of the culture medium 2S) is relatively small, and therefore, the amount of liquid sent by the liquid sending pump 23 (discharge flow rate of the discharged culture solution 2a) is also relatively small. By balancing the amount sent by the liquid sending pumps 13, 23, an amount of liquid in the culture tank 10 can be maintained constant. Moreover, since the gas-utilizing microorganisms 9 are contained in the discharged culture solution 2a, the gas-utilizing microorganisms 9 are discharged from the culture tank 10 together with the culture solution 2a. However, since the gas-utilizing microorganisms 9 grow in the culture solution 2 in the culture tank 10, the concentration of the gas-utilizing microorganisms 9 in the culture solution 2 may be maintained generally constant.

### <Discharge Amount Controlling Step>

A supply situation of the substrate gas and constituents thereof from the substrate gas source 3 depends on a kind and amount of wastes or the like and tends to be unstable. Specifically, a supply flow rate of the substrate gas varies depending on the kind and amount of wastes to be burned. Moreover, a composition (constituents, partial pressure of each constituent or the like) of the substrate gas varies depending on the kind or the like of the wastes to be burned. To cope with this situation, the flow meter 32 and the gas sensor 33 are provided to monitor the supply situation of the substrate gas therewith. A discharge amount of the culture solution 2a from the culture tank 10 may be controlled according to the supply situation of the substrate gas or the predetermined constituent thereof (CO, H₂ or the like).

Specifically, the supply flow rate of the substrate gas is measured with the flow meter 32, for example. Partial pressures of the predetermined constituent of the substrate gas (CO, H₂ or the like) are measured with the gas sensor 33. Based on results of the measurements, supply flow rates of the predetermined constituent to the culture tank 10 are determined. When the supply flow rates of the predetermined constituent (CO, H₂ or the like) are greater than predetermined values (during normal operation), the amount of liquid sent by the liquid sending pumps 13, 23 are maintained low as mentioned above.

### (2) Rapidly-Diluting (Rapidly-Discharging) Operation Mode

On the other hand, when the supply flow rates of the predetermined constituent (CO, H₂ or the like) are equal to or lower than the predetermined values (when substrate gas supply is abnormal), the amount of liquid sent by the liquid sending pump 13 is increased and the amount of liquid sent by the liquid sending pump 23 is also increased to keep balance with the amount of liquid sent by the liquid sending pump 13. Thereby, the culture solution 2a is rapidly discharged to the discharge passage 22 from the discharge port 10e of the culture tank 10 in an amount greater than in the normal operation mode.

Alternatively, when it is known that the supply flow rate of the substrate gas or the predetermined constituent (CO, H₂ or the like) of the substrate gas from the substrate gas source 3 will be decreased at some point in the future due to conditions of waste disposal, a portion 2a of the culture solution 2 in the culture tank 10 may be rapidly discharged from the discharge port 10e beforehand slightly before that point of time.

After the culture solution 2a is rapidly discharged in an amount corresponding to the decrease in the supply flow rate, it is preferable that the discharge flow rate of the culture solution 2a may be returned to generally the same rate as in the normal operation. The point is that it is preferable to discharge a large quantity of the culture solution 2a at one time when the abnormal supply of the substrate gas occurred or before it occurs beforehand instead of continuously discharging a large quantity of the culture solution 2a.

By the rapid discharge of the culture solution 2a, the gas-utilizing microorganisms 9 in the culture solution 2a are also rapidly discharged from the culture tank 10. Therefore, the population of the gas-utilizing microorganisms 9 in the culture tank 10 is decreased. By this arrangement, an amount of the substrate gas that each of the gas-utilizing microorganisms 9 intakes can be secured. Thus, uniform weakening of all of the gas-utilizing microorganisms 9 can be prevented and thereby, death of an entire population of the gas-utilizing microorganisms 9 can be avoided. The culture medium 2S is newly replenished from the culture medium source 12 to the culture tank 10 via the culture medium supply passage 14 (rapid replenishment passage). Preferably, the culture medium 2S is replenished in an amount corresponding to the discharged amount. Thereby, nutrient contents such as vitamins and minerals can be replenished, and nutritional intake of the gas-utilizing microorganisms 9 can be sufficiently secured. As a result, the gas-utilizing microorganisms 9 can be stably cultured. When the supply flow rate of the substrate gas or the predetermined constituent_(CO, H₂ or the like) of the substrate gas is returned to normal, the fermentation by the gas-utilizing microorganisms 9 rapidly revives and the growth of the gas-utilizing microorganisms 9 progresses.

Alternatively, the discharge amount of the culture solution 2a may be maintained at the increased amount from the start of the rapidly discharging till the end of culturing. **In** this case, it is preferable that the amount of replenishment of the culture medium 2S may also be maintained at the increased amount till the end of culturing. The gas-utilizing microorganisms' growing in this environment allows the concentration of the gas-utilizing microorganisms to be kept stable at a value lower than the start of the rapidly discharging.

### <Storing Step>

The discharged culture solution 2a is once stored in the storage tank 84 for extraction via the discharge passage 22 in the normal operation mode and in the rapidly-diluting operation mode (when substrate gas supply is abnormal). The discharged culture solution 2a contains biomass composed of living bodies and dead bodies of the gas-utilizing microorganisms 9 or the like and valuable materials such as ethanol produced by the fermentation in the culture tank 10. By making a capacity of the storage tank 84 for extraction sufficiently large, overflowing of the culture solution 2a from the storage tank 84 for extraction can be prevented even if the discharge flow rate of the culture solution 2a is great when substrate gas supply is abnormal.

### <Extracting Step>

A portion of the culture solution 2a in the storage tank 84 for extraction is introduced to the distillation tower 80 via the send-out passage 81. The portion of the culture solution 2a is distilled in the distillation tower 80, and ethanol (valuable material) is extracted. The ethanol is sent out to the extracted liquid passage 82 from the upper end portion of the distillation tower 80 and provided for various uses via a refining step or the like.

Storing the discharged culture solution 2a once in the storage tank 84 for extraction allows the extraction to be performed as appropriate according to a processing capacity of the distillation tower 80 and a demand for ethanol or the like, thereby avoiding the capacity of the distillation tower 80 being exceeded.

### <Discharge Solution Treating Step>

Extraction residual liquid 2d deposits on the bottom portion of the distillation tower 80. The extraction residual liquid 2d contains the biomass in high concentration. The extraction residual liquid 2d is sent out to the discharge solution treatment part 8 from a lower end portion of the distillation tower 80 via the discharge passage 83. The extraction residual liquid 2d is anaerobically treated or aerobically treated in the discharge solution treatment part 8, and thereby the biomass is degraded. Alternatively, the biomass may be separated and utilized as a fuel (heat source) in the distillation tower 80 or the like.

Other embodiments of the present invention will be described hereinafter. Same reference numerals are used in the drawings to designate parts that correspond to those in foregoing embodiments and description thereof will be omitted.

### <Second Embodiment>

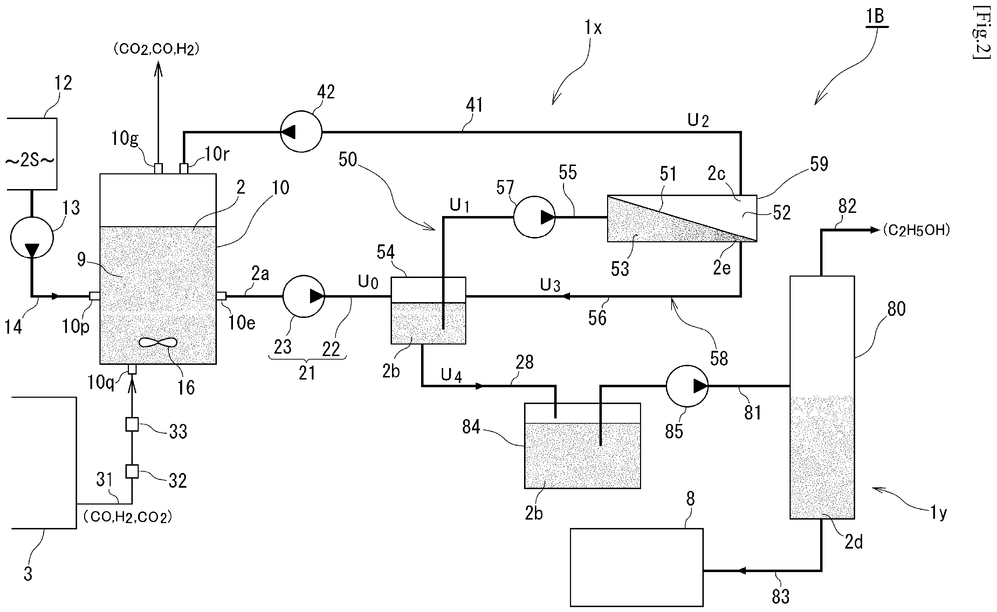

FIG. 2 shows a second embodiment of the present invention. A valuable materials generating system 1B of the second embodiment includes a separation part 50 (biomass concentration part). The separation part 50 includes a filter unit 59, a storage tank 54 for concentration and a circulation passage 58. The filter unit 59 includes a filter (separation film) 51. The filter 51 is made from hollow fibers. An inside of the filter unit 59 is divided into a permeation chamber 52 and a non-permeation chamber 53 by the filter 51.

A culture tank 10 and the storage tank 54 for concentration are connected via a discharge passage 22. A discharged culture solution 2a from the culture tank 10 is stored as a moderately-concentrated culture solution 2b in the storage tank 54 for concentration. The storage tank 54 for concentration and a storage tank 84 for extraction are connected via a send-out passage 28.

The circulation passage 58 includes an outward passage 55 and a return passage 56 (return passage for re-concentration). The storage tank 54 for concentration and the filter unit 59 are connected via the outward passage 55 and the return passage 56. In other words, the storage tank 54 for concentration and the filter unit 59 are disposed in the circulation passage 58. The outward passage 55 is drawn from an inside of the moderately-concentrated culture solution 2b in the storage tank 54 for concentration and continues to an inlet port of the non-permeation chamber 53 of the filter unit 59. A liquid sending pump 57 is disposed in an intermediate portion of the outward passage 55. The return passage 56 extends from an outlet port of the non-permeation chamber 53 and continues to the storage tank 54 for concentration.

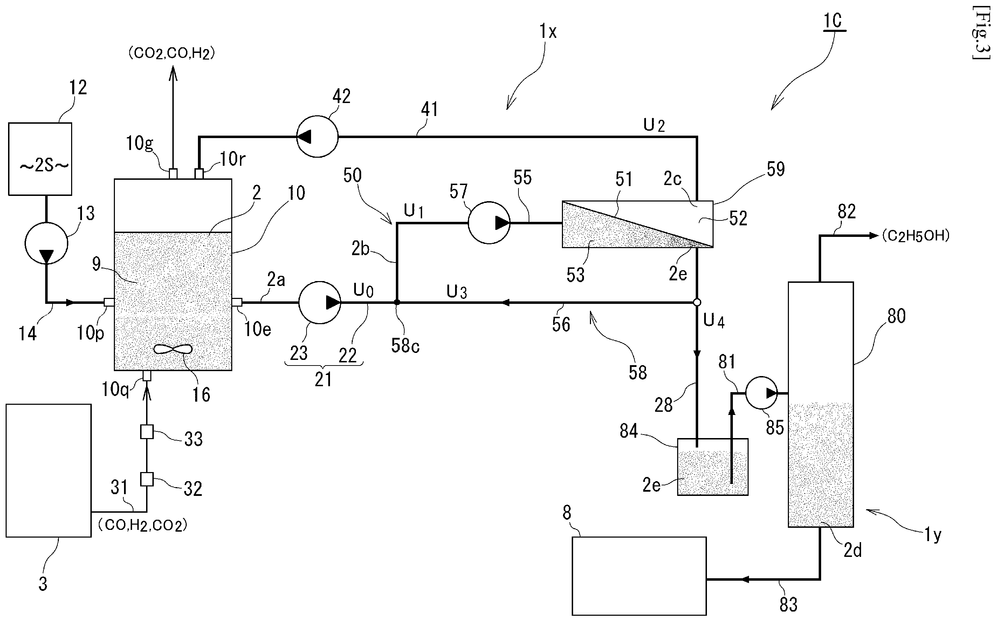

The storage tank 54 for concentration may not have a large capacity. When a total capacity of the outward passage 55, the return passage 56 and the non-permeation chamber 53 is greater than a certain capacity, the storage tank 54 for concentration may be omitted (see FIG. 3).

The filter unit 59 and the culture tank 10 are connected via a diluted solution return passage 41. The diluted solution return passage 41 extends from an outlet port of the permeation chamber 52 and continues to a return port 10r of the culture tank 10. A liquid return pump 42 is disposed in the diluted solution return passage 41. Pressure fluctuation of the liquid return pump 42 can be constrained by providing the storage tank 54 for concentration.

In the valuable materials generating system 1B, the discharged culture solution 2a from the culture tank 10 is once stored as the culture solution 2b in the storage tank 54 for concentration. The culture solution 2b is circulated between the storage tank 54 for concentration and the filter unit 59, and thereby gas-utilizing microorganisms 9 are concentrated. Specifically, the culture solution 2b is introduced to the non-permeation chamber 53 of the filter unit 59 by activation of the liquid sending pump 57. Liquid constituents in the non-permeation chamber 53 can permeate the filter 51 and move to the permeation chamber 52. On the other hand, solid constituents of the liquid including the biomass composed of living bodies and dead bodies of the gas-utilizing microorganisms 9 or the like are prohibited from penetrating the filter 51. Therefore, the moderately-concentrated culture solution 2b is separated into a diluted culture solution 2c in the permeation chamber 52 and a concentrated culture solution 2e in the non-permeation chamber 53. A biomass concentration of the diluted culture solution 2c is sufficiently lower than that of the culture solution 2 in the culture tank 10. Preferably, the diluted culture solution 2c hardly contains biomass. A biomass concentration of the concentrated culture solution 2e is higher than that of the culture solution 2 in the culture tank 10. Thus, the diluted culture solution 2c is a biomass-diluted culture solution in which the biomass is diluted (including complete removal). The concentrated culture solution 2e is a biomass-highly-concentrated culture solution in which the biomass is highly concentrated.

The diluted culture solution 2c is returned to the culture tank 10 via the diluted solution return passage 41 by driving the liquid return pump 42. A supply flow rate of a culture medium 2S can be decreased by a flow rate corresponding to a return flow rate at which the diluted culture solution 2c is returned to the culture tank 10, thereby, a waste of the culture solution can be reduced. Moreover, when the supply of substrate gas is abnormal, increase in the supply flow rate of the culture medium 2S can be constrained by returning more diluted culture solution 2c to the culture tank 10 than in normal operation. Therefore, change in composition of liquid constituents of the culture solution 2 is small, thereby the probability of the gas-utilizing microorganisms 9 dying of shock can be reduced.

Preferably, the biomass concentration of the diluted culture solution 2c in the filter unit 59 is sufficiently low, more preferably almost zero, when the capacity of the storage tank 54 for concentration is great and a retaining time of the culture solution 2b in the tank 54 is long. By this arrangement, even if the gas-utilizing microorganisms 9 died in the culture solution 2b, interfusion of dead bodies of the gas-utilizing microorganisms 9 into the culture tank 10 with the diluted culture solution 2c can be prevented.

The composition of the liquid constituents of the culture solution 2 in the culture tank 10 may not be the same as a composition of the culture medium 2S. Life activities of the gas-utilizing microorganisms 9 may consume or produce some liquid constituents. Therefore, if a supplied amount of the culture medium 2S is excessively great, it is possible that the gas-utilizing microorganisms 9 may die of shock due to a sudden change in the environment.

The concentrated culture solution 2e is returned to the storage tank 54 for concentration via the return passage 56. Therefore, the moderately-concentrated culture solution 2b in the storage tank 54 for concentration is a mixture of the discharged culture solution 2a and the concentrated culture solution 2e (biomass-highly-concentrated culture solution). The moderately-concentrated culture solution 2b has a higher biomass concentration (concentration of gas-utilizing microorganisms) than the culture solutions 2, 2a. Specifically, the biomass including living bodies and dead bodies of the gas-utilizing microorganisms 9 is more concentrated in the moderately-concentrated culture solution 2b than in the culture solutions 2, 2a. A portion of the moderately-concentrated culture solution 2b in the storage tank 54 for concentration is stored in the storage tank 84 for extraction via the send-out passage 28. Then the portion of the moderately-concentrated culture solution 2b is sent out to a distillation tower 80 from the storage tank 84 for extraction, provided for extraction of ethanol, and further provided for a discharge solution treating step in a discharge solution treatment part 8.

The culture solution 2a rapidly discharged when the supply of substrate gas is abnormal can be stored in the storage tank 84 for extraction via the storage tank 54 for concentration.

A sending-out flow rate U₄ from the storage tank 54 for concentration to the storage tank 84 for extraction is kept at a rate smaller than a discharge flow rate U₀ of the culture solution 2a (U₀>U₄). This allows a stored amount of the moderately-concentrated culture solution 2b in the storage tank 54 for concentration to be secured. The biomass concentration in the moderately-concentrated culture solution 2b is U₀/U₄ times the biomass concentration in the culture solutions 2, 2a.

Moreover, a sending-out flow rate U₁ of the moderately-concentrated culture solution 2b to the filter unit 59 is kept at a rate greater than the discharge flow rate U₀ of the culture solution 2a (U₁>U₀) by controlling outputs of the pumps 23, 42, 57. Preferably, U₁ is in a general range of from U₁=2×U₀ to U₁=100×U₀. This allows a flow in the non-permeation chamber 53 to be great, thereby preventing the filter 51 from being clogged. The flow rate U₃ of the concentrated culture solution 2e is U₃=U₁-U₂.

### <Third Embodiment>

The storage tank 54 for concentration shown in FIG. 2 may be omitted. Of a filter unit 59 and the storage tank 54 for concentration, at least the filter unit 59 should be disposed in a circulation passage 58.

As shown in FIG. 3, in a valuable materials generating system 1C of a third embodiment, the storage tank 54 for concentration in the second embodiment (FIG. 2) is omitted and a confluent portion 58c is disposed in place of the storage tank 54 for concentration. A discharge passage 22 and a return passage 56 are directly (not via the storage tank 54 for concentration) in the confluent portion 58c. An outward passage 55 extends from the confluent portion 58c. A send-out passage 28 is branched from an intermediate portion of the return passage 56. The send-out passage 28 is connected to a storage tank 84 for extraction.

### (1) Normal Operation Mode

A discharged culture solution 2a from a culture tank 10 flows in the discharge passage 22 and is mixed with a culture solution 2e from the return passage 56 in the confluent portion 58c. The mixed culture solution is circulated in the circulation passage 58 in an order of from the outward passage 55 to the filter unit 59 and to the return passage 56. In the filter unit 59, the mixed culture solution is separated into a diluted culture solution 2c and a concentrated culture solution 2e. The diluted culture solution 2c is returned to the culture tank 10 via a diluted solution return passage 41. A portion of the concentrated culture solution 2e is branched to the send-out passage 28 and stored in the storage tank 84 for extraction and then provided for ethanol extraction in a distillation tower 80. A remainder of the concentrated culture solution 2e is flown to the confluent portion 58c via the return passage 56.

The relations among flow rates U₀, U₁, U₂, U₃ and U₄ at the passages 22, 55, 41, 56 and 28 are similar to those in the second embodiment (FIG. 2). Therefore, circulating flow rates U₁, U₃ of the culture solutions 2b, 2e in the circulation passage 58 are sufficiently greater than a discharge flow rate U₀ of the discharged culture solution 2a (U₁>U₀, U₃> U₀).

### (2) Rapidly-Diluting Operation Mode

When a supply flow rate of a substrate gas (or predetermined constituent thereof) is decreased to an abnormal degree, the discharge flow rate of the discharged culture solution 2a from the culture tank 10 is temporarily increased. The discharged culture solution 2a is sent out to the storage tank 84 for extraction via the passages 22, 55, 53 and 28 in this order or via a bypass passage that is not shown in the drawings. The diluted culture solution 2c that permeated through a filter 51 and a culture medium 2S from the culture medium source 12 are replenished to the culture tank 10 in an amount corresponding to the discharged amount of the discharged culture solution 2a. By this arrangement, a density of gas-utilizing microorganisms 9 in the culture tank 10 can be reduced and even when the supply flow rate of the substrate gas is decreased, the gas-utilizing microorganisms 9 can be stably cultured.

### <Fourth Embodiment>

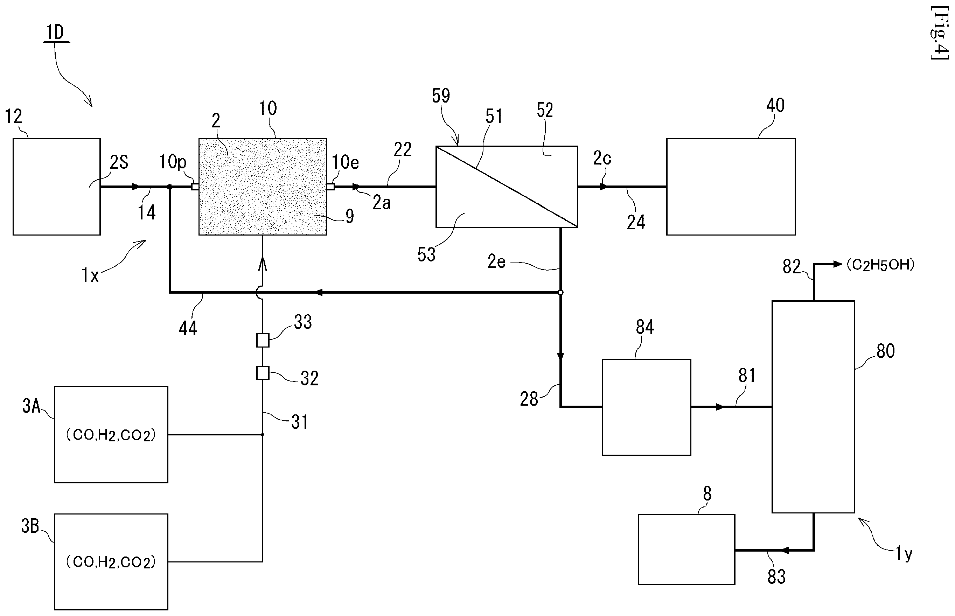

FIGS. 4 to 7 show a fourth embodiment of the present invention.

As shown in FIG. 4, a culture apparatus 1x of a valuable materials generating system 1D includes a culture tank 10, a culture medium source 12, a filter unit 59 and a diluted solution storage tank 40. Two (plurality of) substrate gas sources 3A, 3B are connected to the culture tank 10 via a gas supply passage 31.

As shown in FIGS. 4 to 7, the valuable materials generating system 1D is run in four operation modes according to supply situations of a substrate gas or predetermined constituent thereof (CO, H₂ or the like). Connection relationships among components 10, 12, 59, 40, 84 of the system 1D vary according to the operation modes.

In the actual system 1D, the components 10, 12, 59, 40, 84 are connected by piping such that they can accommodate connection variations for all the operation modes. Circuit configuration is changed by opening and closing some valves of piping and/or turning on and off liquid sending pumps according to the operation mode. For ease of understanding, only piping lines that are open are depicted in the drawings for each operation mode. Valves and pumps are not shown in the drawings.

### (1) Normal Operation Mode

As shown in FIG. 4, when the supply situations of the substrate gas from the two substrate gas sources 3A, 3B are normal, the system 1D is run in a normal operation mode. In the normal operation mode, the culture medium source 12 is connected to the culture tank 10 and the culture tank 10 is connected to the filter unit 59.

An outlet port of a non-permeation chamber 53 is connected to a storage tank 84 for extraction (breeding tank) via a concentrated solution send-out passage 28. The outlet port is also connected to a culture medium supply port 10p of the culture tank 10 via a concentrated solution return passage 44.

An outlet port of a permeation chamber 52 is connected to the diluted solution storage tank 40 (permeate tank) via a diluted solution storage passage 24.

Preferably, a capacity of the diluted solution storage tank 40 is equal to or greater than a capacity of the culture tank 10. Thereby, gas-utilizing microorganisms 9 in the culture tank 10 can be diluted to any concentration in a rapidly-diluting operation mode to be described later.

In the normal operation mode, a constant flow of a culture medium 2S is supplied from the culture medium source 12 to the culture tank 10 and the gas-utilizing microorganisms 9 are cultured in a culture solution 2 in the culture tank 10 (culturing step).

A certain flow rate of a discharged culture solution 2a is discharged from the culture tank 10 (discharging step). The discharged culture solution 2a is separated into a diluted culture solution 2c and a concentrated culture solution 2e at the filter unit 59 (separating step). A filter 51 of the filter unit 59 may be a UF (ultra-filtration) film of a cross-flow type, for example.

A circulation passage 58 similar to those in the second and the third embodiments (FIGS. 2 and 3) may be disposed and the filter unit 59 may be disposed in the circulation passage 58.

Preferably, a biomass concentration of the gas-utilizing microorganisms 9 or the like in the diluted culture solution 2c (filtrated solution) from the filter unit 59 may be generally zero. The diluted culture solution 2c is stored in the diluted solution storage tank 40 through the diluted solution storage passage 24 (diluted solution storing step). Composition of the diluted culture solution 2c in the diluted solution storage tank 40 is generally the same as a composition of liquid constituents in the culture tank 10.

A portion of the concentrated culture solution 2e from the non-permeation chamber 53 is stored in the storage tank 84 for extraction through the concentrated solution send-out passage 28. The concentrated culture solution 2e in the storage tank 84 for extraction is sent out to a distillation tower 80 and provided for extraction of ethanol.

A remainder (preferably a large part) of the concentrated culture solution 2e from the non-permeation chamber 53 is returned to the culture tank 10 via the concentrated solution return passage 44 with the culture medium 2S (fresh media) from the culture medium source 12. Accordingly, in total, a discharge speed of the gas-utilizing microorganisms 9 in the culture solution 2 in the culture tank 10 is slower than a discharge speed of the liquid constituents of the culture solution 2, and the biomass concentration of the culture solution 2 is maintained high. By balancing a total of a return flow rate of the concentrated culture solution 2e and a supply flow rate of the culture medium 2S with a discharge flow rate of the discharged culture solution 2a, an amount of the culture solution 2 in the culture tank 10 can be maintained constant.

### (2) Rapidly-Diluting (Rapidly-Discharging) Operation Mode

Let us assume that one of the substrate gas sources 3A, 3B (substrate gas source 3A, for example) stops producing the substrate gas due to a trouble such as a failure. In this case, a supply flow rate of the substrate gas is reduced to a half of that in the normal operation mode. In other words, since there are two (plurality of) the substrate gas sources 3A, 3B, gas supply in half the amount can be secured even if one of the substrate gas sources 3A, 3B stops.

When a supplied amount of the substrate gas is not sufficient, the gas-utilizing microorganisms 9 in the culture tank 10 may be dead or change metabolism so as to survive under a small quantity of gas. When the metabolism is changed, desired constituents such as ethanol may not be produced and by-products such as acetic acid may be produced in a large quantity. The metabolism may not be easily returned to the original even if the supplied amount of gas is recovered, which may damage the production of ethanol to a great extent.

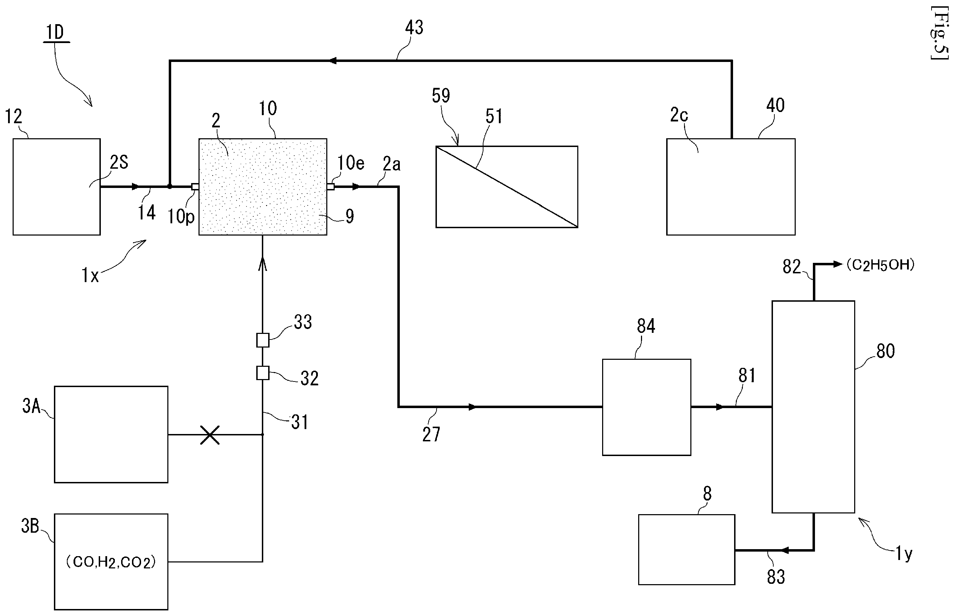

To prevent such a situation, the system is run in a rapidly-diluting operation mode as shown in FIG. 5. In the rapidly-diluting operation mode, a discharge port 10e of the culture tank 10 and the storage tank 84 for extraction are directly communicated via a rapid discharge passage 27 (discharge control part). The diluted solution storage tank 40 and the supply port 10p of the culture tank 10 are communicated via a rapid replenishment passage 43.

Then a discharge amount of the discharged culture solution 2a from the culture tank 10 is temporarily increased. In other words, the culture solution 2 is rapidly discharged from the culture tank 10 (rapidly-discharging step). By this arrangement, the population of the gas-utilizing microorganisms 9 in the culture tank 10 is reduced. Preferably, a degree of reduction of the gas-utilizing microorganisms 9 is determined according to a degree of decrease of the supply flow rate of the substrate gas. In this example, the supply flow rate of the substrate gas is halved. Therefore, about a half of the culture solution 2 is discharged to reduce the population of the gas-utilizing microorganisms 9 in the culture tank 10 to the half. By this arrangement, an amount of the substrate gas that each of the gas-utilizing microorganisms 9 intakes can be maintained at a generally the same amount as in the normal operation mode. As a result, uniform weakening of all of the gas-utilizing microorganisms 9 and thereby, death of the entire population of the gas-utilizing microorganisms 9 can be avoided.

The discharged culture solution 2a rapidly discharged from the culture tank 10 is sent out to the storage tank 84 for extraction via the rapid discharge passage 27.

The rapid discharge passage 27 may pass through the non-permeation chamber 53 of the filter unit 59.

At the same time with the rapid discharge (increase in the discharge amount) of the discharged culture solution 2a, the diluted culture solution 2c in the diluted solution storage tank 40 is returned to the culture tank 10 via the rapid replenishment passage 43 (stored diluted solution rapidly replenishing step). Accordingly, there is no need to increase the supply flow rate of the culture medium 2S. A concentration of the gas-utilizing microorganisms in the culture solution 2 in the culture tank 10 can be diluted by the rapid replenishment of the diluted culture solution 2c.

The diluted culture solution 2c has generally the same composition as the liquid constituents of the culture solution 2. Accordingly, even if the diluted culture solution 2c is supplied to the culture tank 10 in a large quantity, it will not cause a rapid change in a liquid composition of the culture solution 2. Therefore, the gas-utilizing microorganisms 9 can be prevented from being damaged by the rapid change in the liquid composition. By maintaining the biomass concentration of the diluted culture solution 2c at generally zero, interfusion of dead bodies or the like of the gas-utilizing microorganisms 9 into the culture tank 10 can be prevented.

Alternatively, even if the biomass concentration of the diluted culture solution 2c from the filter unit 59 (filtrated solution) is not zero, the biomass may become deposited during a storage period in the diluted solution storage tank 40. Thereby, the biomass may be separated from supernatant liquid of the diluted culture solution 2c, and the supernatant liquid may be rapidly replenished to the culture tank 10 in the rapidly-diluting operation mode.

A concentration of ethanol in the discharged culture solution 2a can be prevented from becoming thinner. Accordingly, increase in load during distillation or the like in the distillation tower 80 can be prevented and an efficiency of ethanol extraction can be maintained high. Moreover, a greater amount of the diluted culture solution 2c can be returned to the culture tank 10 in a shorter period of time compared with a case where the diluted culture solution 2c is directly returned from the permeation chamber 52 to the culture tank 10 (refer to the second embodiment (FIG. 2) and the third embodiment (FIG. 3)). Moreover, the filter 51 can be downsized, and thereby, a building cost can be constrained.

If the diluted culture solution 2c were directly returned from the permeation chamber 52 to the culture tank 10, the filter 51 would have to be upsized for securing rapid discharge of a large amount of the discharged culture solution 2a (massive permeation of the filter 51), thereby increasing the construction cost.

Even if the discharged culture solution 2a is discharged in a large amount from the culture tank 10, an amount of stored solution in the culture tank 10 can be maintained constant by returning the diluted culture solution 2c in the diluted solution storage tank 40 to the culture tank 10. Therefore, a stirrer 16 (see FIGS. 1 to 3) will not be exposed. Even when the culture tank 10 is made of a loop reactor vertically extending lengthwise, circulation failure due to lack of the culture solution 2 can be avoided.

Preferably, the rapidly-diluting operation mode, i.e. a rapidly discharging operation of the discharged culture solution 2a and a rapidly replenishing operation of the diluted culture solution 2c may be terminated after a few minutes to a few dozens of minutes.

### (3) Low Biomass Operation Mode

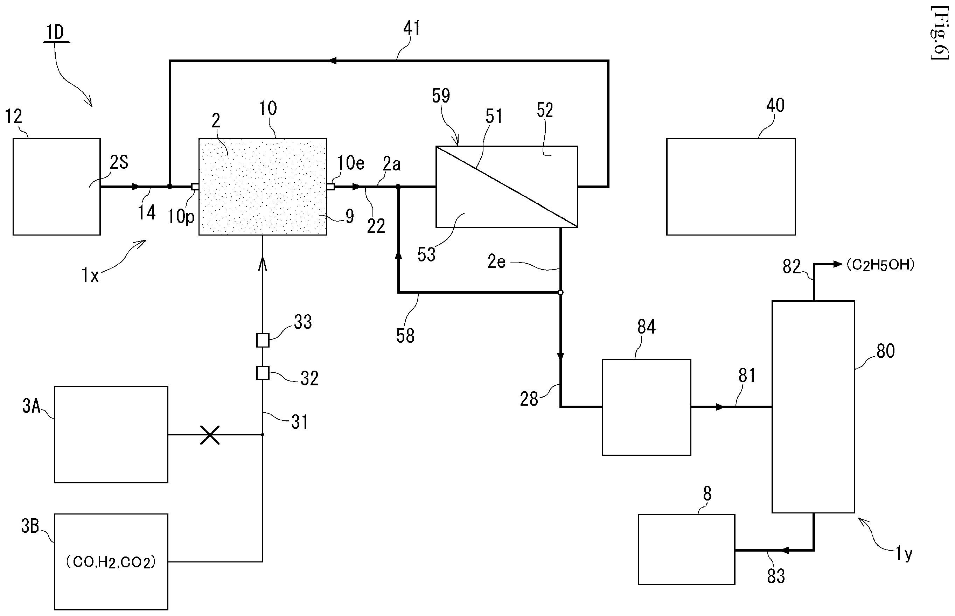

As shown in FIG. 6, the system is run in a low biomass operation mode after the end of the rapidly-diluting operation mode until troubles at the substrate gas source 3A is resolved and the supply flow rate of the substrate gas returns to a predetermined value or higher. In the low biomass operation mode, communication between the permeation chamber 52 and the diluted solution storage tank 40 is blocked and the communication between the diluted solution storage tank 40 and the culture tank 10 is blocked. Instead, the outlet port of the permeation chamber 52 and the supply port 10p of the culture tank 10 are connected by a diluted solution return passage 41. The outlet port and an inlet port of the non-permeation chamber 53 are loop-connected via the circulation passage 58. Accordingly, a circuit configuration of the valuable materials generating system 1D in the rapidly-diluting operation mode is substantially the same as that of the valuable materials generating system 1C in the third embodiment.

The diluted culture solution 2c obtained in the separating step in the filter unit 59 is returned to the culture tank 10 without being sent out to the diluted solution storage tank 40. A portion of the concentrated culture solution 2e obtained in the separating step is returned to the non-permeation chamber 53 via the circulation passage 58 and the remainder of the concentrated culture solution 2e is sent out to the storage tank 84 for extraction and eventually to subsequent apparatus 1y. Accordingly, in total, the discharge speed of the gas-utilizing microorganisms 9 in the culture solution 2 is faster than the discharge speed of the liquid constituents of the culture solution 2. By this arrangement, even if the gas-utilizing microorganisms 9 grow in the culture tank 10, the biomass concentration inside the culture tank 10 can be maintained low by discharging the gas-utilizing microorganisms 9 from the culture tank 10 in an amount corresponding to the grown amount. Therefore, even when the supply flow rate of the substrate gas is low, the gas-utilizing microorganisms 9 can be cultured in a stable manner and the gas-utilizing microorganisms 9 can be prevented from dying and changing metabolism.

### (4) State Recovery Operation Mode

The system is run in a state recovery operation mode after the troubles at the substrate gas source 3A is resolved and the supply flow rate of the substrate gas becomes returnable to the predetermined value or higher.

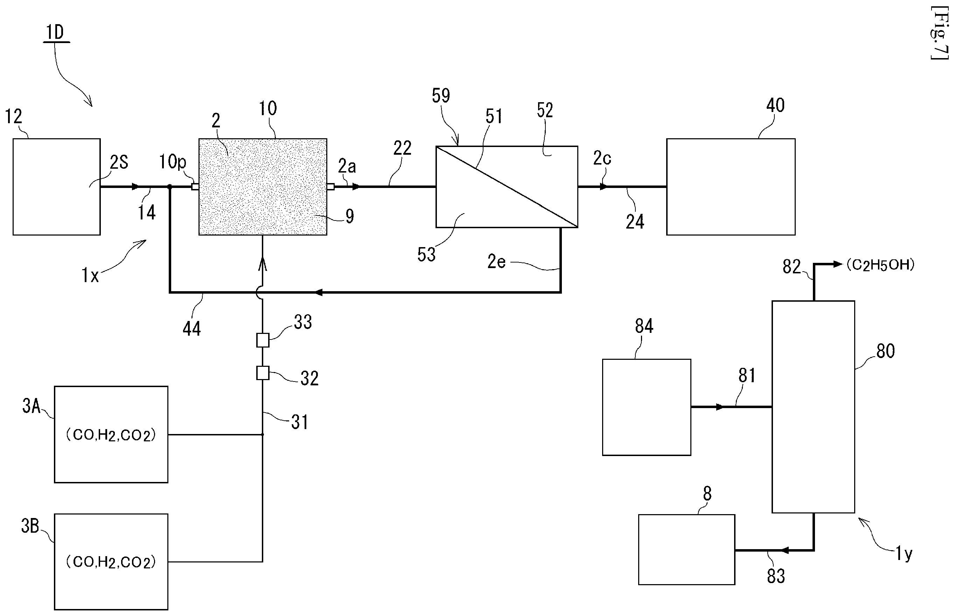

As shown in FIG. 7, in the state recovery operation mode, the permeation chamber 52 and the diluted solution storage tank 40 can be communicated through the diluted solution storage passage 24. Communication between the non-permeation chamber 53 and the storage tank 84 for extraction is blocked. The outlet port of the non-permeation chamber 53 is communicable only with the culture medium supply port 10p of the culture tank 10 through the concentrated solution return passage 44. Accordingly, a whole amount of the concentrated culture solution 2e obtained in the separating step in the filter unit 59 is returned to the culture tank 10 through the concentrated solution return passage 44. A length of time between a time when the culture solution 2b is discharged from the culture tank 10 to a time when the culture solution 2b is returned to the culture tank 10 as the concentrated culture solution 2e should be a length of time in which the gas-utilizing microorganisms 9 can live without the substrate gas, which is preferably 1 minute or shorter, and 3 hours or shorter at the longest.

The whole amount of the diluted culture solution 2c is stored in the diluted solution storage tank 40.

Monitoring a condition (concentration of required constituent) of the culture solution 2 in the culture tank 10, the supply flow rate of the substrate gas to the culture tank 10 is increased. This causes the gas-utilizing microorganisms 9 in the culture solution 2 to grow; thereby the concentration of the gas-utilizing microorganisms 9 can be rapidly increased.

Accompanying the increase in the concentration of the gas-utilizing microorganisms 9, the supply flow rate of the substrate gas is increased. Preferably, the supply flow rate of the substrate gas is proportional to the concentration of the gas-utilizing microorganisms 9. An amount of supply of the culture medium 2S (fresh media) from the culture medium source 12 is controlled so that the liquid composition (such as a concentration of acetic acid) of the culture solution 2 may be maintained stable. When the concentration of the gas-utilizing microorganisms 9 and the supply flow rate of the substrate gas reach predetermined values, the operation may be switched to the normal operation mode by making the non-permeation chamber 53 and the storage tank 84 for extraction communicable with each other as shown in FIG. 4.

### <Fifth Embodiment>

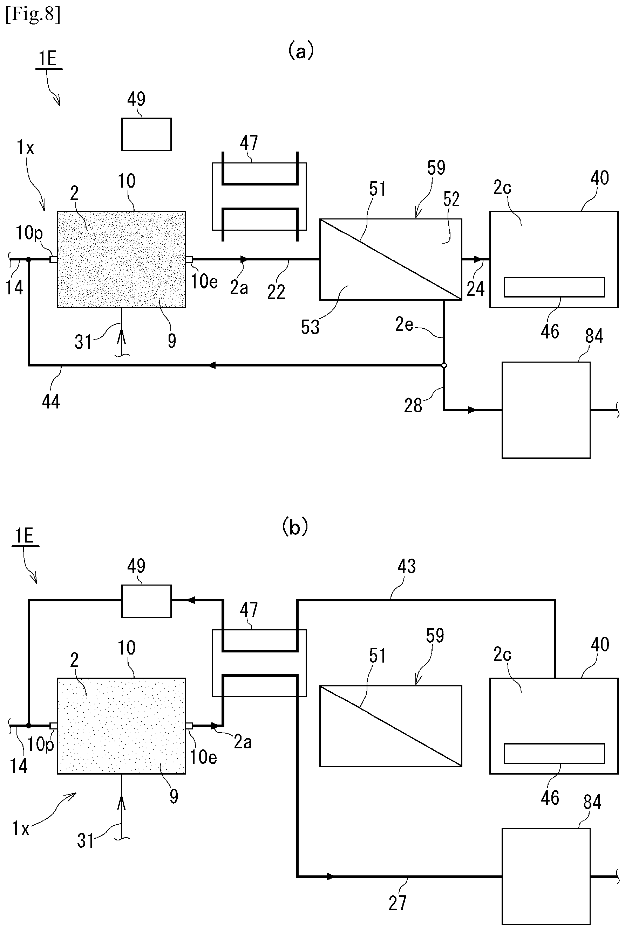

FIG. 8 shows a fifth embodiment of the present invention.

As shown in FIG. 8 (a), a cooler 46 (liquid temperature conditioner) is disposed in a diluted solution storage tank 40 of a valuable materials generating system 1E. As shown in FIG. 8 (b), a heat exchanger 47 is disposed between a rapid replenishment passage 43 and a rapid discharge passage 27 of the system 1E in a rapidly-diluting operation mode. Moreover, a heater 49 is disposed in the rapid replenishment passage 43 at a location closer to a culture tank 10 than the heat exchanger 47.

### (1) Normal Operation Mode

As shown in FIG. 8 (a), in the valuable materials generating system 1E, a diluted culture solution 2c stored in the diluted solution storage tank 40 is cooled in the cooler 46 to make a temperature of the diluted culture solution 2c lower than a temperature of the culture tank 10. Preferably, a temperature of the cooler 46 may be set at a temperature at which organisms such as bacteria cannot survive or a temperature at which life activities such as metabolism and breeding can be rendered impossible or constrained and a temperature at which the diluted culture solution 2c does not freeze. In this embodiment, the temperature is set at 0 to 20 °C, for example, and preferably set at around 4 °C. Thereby, breeding of putrefying bacteria in the diluted culture solution 2c can be constrained or prevented and generation of odor can be prevented.

### (2) Rapidly-Diluting Operation Mode

As shown in FIG. 8(b), in the rapidly-diluting operation mode, heat is exchanged between the diluted culture solution 2c that passes through the rapid replenishment passage 43 and a discharged culture solution 2a that passes through the rapid discharge passage 27 in the heat exchanger 47. By the heat exchange, the diluted culture solution 2c may be heated to a temperature close to that of the culture tank 10. By utilizing the discharged culture solution 2a as a heat source, load to the heater 49 to be described later can be reduced.

After that, the diluted culture solution 2c is heated further by the heater 49. Thereby, the temperature of the diluted culture solution 2c can be sufficiently close to a temperature of a culture solution 2 in the culture tank 10, and preferably, generally the same as the temperature of the culture solution 2. After that, the diluted culture solution 2c is provided to the culture tank 10 and mixed with the culture solution 2. By this arrangement, even when a large amount of the diluted culture solution 2c is provided, a change in a liquid temperature of the culture solution 2 can be sufficiently constrained. Therefore, gas-utilizing microorganisms 9 in the culture tank 10 can be prevented from being damaged due to the change in the liquid temperature.

A heater may be used as a liquid temperature conditioner for the diluted solution storage tank 40 in place of the cooler 46. By this heater, the diluted culture solution 2c in the diluted solution storage tank 40 may be heated to a temperature higher than that of the culture tank 10. Preferably, temperature settings for the heating may be set at 50 to 100 °C. By setting the temperature at 50°C or higher, it is possible to make organisms such as bacteria unable to survive or to make life activities such as metabolism and breeding impossible or constrained. By setting the temperature at lower than 100°C, boiling of the diluted culture solution 2c and denaturation of constituents of the diluted culture solution 2c can be prevented.

When a heater is provided in the diluted solution storage tank 40, it is preferable to provide a cooler instead of the heater 49 at the rapid replenishment passage 43 between the heat exchanger 47 and the culture tank 10.

### <Sixth Embodiment>

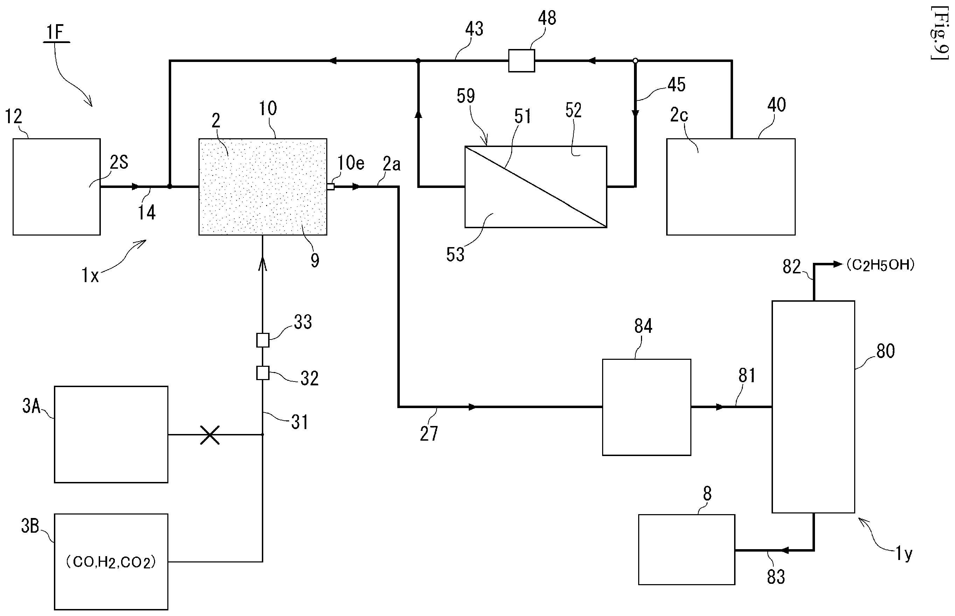

FIG. 9 shows a sixth embodiment of the present invention, which is a modified embodiment of the rapidly-diluting operation mode.

As shown in FIG. 9, in a valuable material generating system 1F in a rapidly-diluting operation mode, a backwash passage 45 is branched from a rapid replenishment passage 43. The backwash passage 45 passes through a filter unit 59 in an order of from a permeation chamber 52 to a non-permeation chamber 53 and joins the rapid replenishment passage 43 again. A flow rate control valve 48 is disposed in the rapid replenishment passage 43 at a portion between a point where the backwash passage 45 branches therefrom and a point where the backwash passage 45 joins the replenishment passage 43.

In the rapidly-diluting operation mode, a portion of a diluted culture solution 2c from a diluted solution storage tank 40 is branched from the rapid replenishment passage 43 to the backwash passage 45 and flows backward in the filter unit 59. Thereby, a filter 51 can be backwashed and clogging of the filter 51 may be reduced or removed. The diluted culture solution 2c after backwashing joins the diluted culture solution 2c that flowed forward in the rapid replenishment passage 43 and is led into a culture tank 10. By using only a portion of the diluted culture solution 2c for backwashing, a flow rate of the diluted culture solution 2c as a whole can be secured. A flow rate for backwashing can be controlled by the flow rate control valve 48.

Alternatively, the entirety of the diluted culture solution 2c may backwash the filter unit 59. An on-off valve may be disposed in the rapid replenishment passage 43 in place of the flow rate control valve 48.

### <Seventh Embodiment>

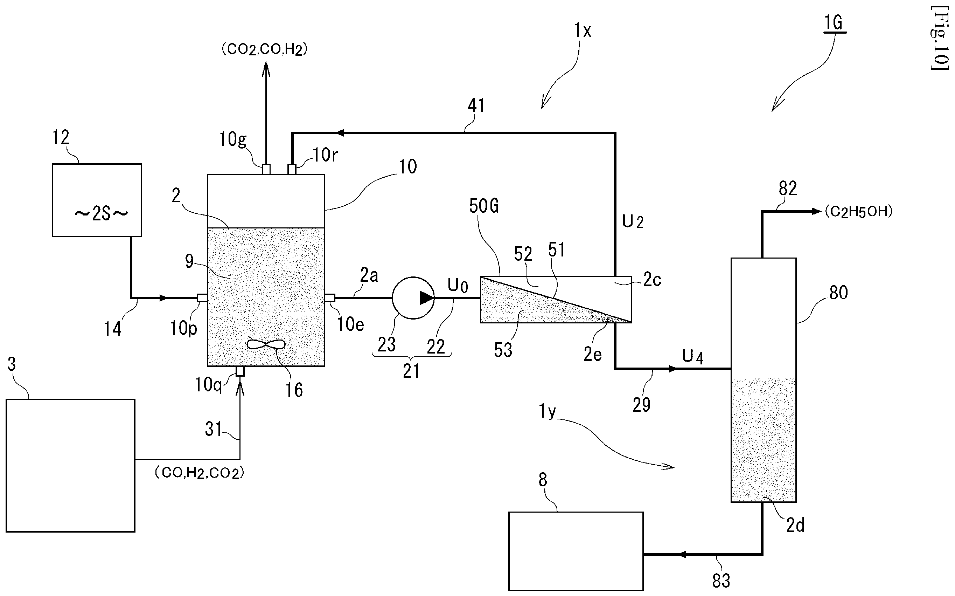

FIG. 10 shows a seventh embodiment of the present invention. A valuable materials generating system 1G includes a culture apparatus 1x and subsequent apparatus 1y. The culture apparatus 1x includes a culture tank 10 and a biomass concentration part 50G (separation part). The subsequent apparatus 1y includes a distillation tower 80 (extraction part) and a discharge solution treatment part 8.

A culture solution 2 is stored in the culture tank 10. Gas-utilizing microorganisms 9 are cultured in the culture solution 2. Anaerobic bacteria may be used as the microorganisms 9 as disclosed in the Patent Document 1 (United States Patent Application Publication No. US2013/0065282), Japanese Patent Application Publication No. 2014-050406 and Japanese Patent Application Publication No. 2004-504058, etc. Valuable materials such as ethanol (C₂H₅OH) are synthesized from a substrate gas by fermentation activities of the microorganisms 9. Substrate gas constituents (predetermined constituent) used for the fermentation of the microorganisms 9 may be chiefly carbon monoxide (CO) and hydrogen (H₂). The valuable materials may include ethanol, butanol, ascetic acid or acetate and other organic compounds.

The culture solution 2 in the culture tank 10 is stirred by a stirrer 16. Therefore, the gas-utilizing microorganisms 9 are evenly dispersed throughout the culture solution 2.

A culture medium source 12 is connected to the culture tank 10. A culture medium 2S of the culture solution 2 is stored in the culture medium source 12. In other words, the culture solution 2 before the gas-utilizing microorganisms 9 are put therein is stored. The culture medium 2S is composed mostly of water (H₂O) with nutrient contents such as vitamins and phosphoric acids dispersed or dissolved therein. A culture medium supply passage 14 extends from the culture medium source 12. The culture medium supply passage 14 continues to a culture medium supply port 10p of the culture tank 10.

Moreover, a substrate gas source 3 is connected to the culture tank 10. A gas supply passage 31 extends from the substrate gas source 3. The gas supply passage 31 continues to a gas supply port 10q in a bottom portion of the culture tank 10. Although not shown in detail in the drawing, the substrate gas source 3 may be an industrial waste disposal facility that treats industrial wastes or the like. In other words, the culture apparatus 1x, and thus the valuable materials generating system 1G of this embodiment is incorporated in an industrial waste disposal system. The substrate gas source 3 has a melting furnace. Wastes are burned in the melting furnace by a highly-concentrated oxygen gas and resolved to a low molecular level. Finally, an anaerobic substrate gas including carbon monoxide (CO), hydrogen (H₂) and carbon dioxide (CO₂) is produced. A produced flow rate and composition of the substrate gas are not stable, depending on a kind and amount or the like of wastes.

The biomass concentration part 50G is a separator including a filter 51 (separation film). A hollow-fiber membrane, for example, is used as the filter 51. A permeation chamber 52 and a non-permeation chamber 53 are defined by the filter 51.

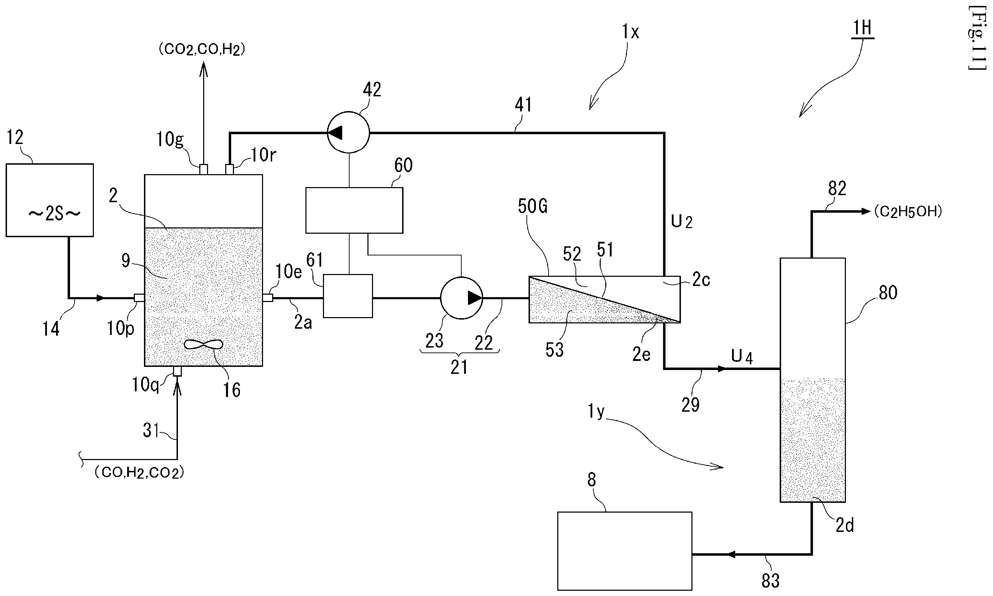

A culture tank 10 and the biomass concentration part 50G are connected via a culture solution discharge passage 22 and a diluted solution return passage 41. The discharge passage 22 extends from a discharge port 10e in an intermediate portion or the bottom portion of the culture tank 10 and continues to an inlet port of the non-permeation chamber 53. A liquid sending pump 23 is disposed in the discharge passage 22. The diluted solution return passage 41 extends from an outlet port of the permeation chamber 52 and continues to a return port 10r of the culture tank 10. A liquid return pump 42 (see FIG. 11) may be disposed in the diluted solution return passage 41.

Moreover, a concentrated solution send-out passage 29 extends from the outlet port of the non-permeation chamber 53 to the subsequent apparatus 1y. The concentrated solution send-out passage 29 continues to a middle portion of the distillation tower 80. An extracted liquid passage 82 extends from an upper end portion of the distillation tower 80. A discharge passage 83 extends from a bottom portion of the distillation tower 80. The discharge passage 83 continues to the discharge solution treatment part 8. While not shown in detail in the drawings, the discharge solution treatment part 8 includes an anaerobic treatment part and an aerobic treatment part for a discharge solution.

A culture method and a method for generating valuable materials by the valuable materials generating system 1G will be described below.

### <Culturing Step>

The culture solution 2 is put in the culture tank 10 and the gas-utilizing microorganisms 9 are cultured in the culture solution 2. The gas-utilizing microorganisms 9 may be evenly dispersed throughout the culture solution 2 by stirring the culture solution 2 with the stirrer 16.

### <Substrate Gas Supplying Step>

The substrate gas (CO, H₂, CO₂ or the like) produced from the wastes in the substrate gas source 3 is introduced to the culture tank 10 via the gas supply passage 31 to dissolve the substrate gas in the culture solution 2 in the culture tank 10. Dissolving of the substrate gas in the culture solution 2 can be promoted by the stirring.

A supply flow rate of the substrate gas from the substrate gas source 3 may not be always stable.

### <Fermentation Step>

In this step, the gas-utilizing microorganisms 9 in the culture solution 2 ferment to produce valuable materials such as ethanol from the substrate gas. Gas constituents such as CO₂ are also produced by the fermentation. Gas constituents such as CO₂ introduced via the gas supply passage 31, CO₂ produced by the fermentation and unused CO, H₂ or the like are discharged from the discharge port 10g of the culture tank 10. These gas constituents may be returned to the substrate gas source 3 or may be burned to be utilized as a heat source for distillation or the like.

### <Discharging Step>

A portion 2a of the culture solution 2 (to be referred to as a "discharged culture solution 2a" hereinafter as appropriate) in the culture tank 10 is discharged to a discharge passage 22 by activating the liquid sending pump 23. The discharged culture solution 2a is sent out to the biomass concentration part 50G via the discharge passage 22. The discharging of the culture solution 2a may be done continuously or intermittently. The discharging of the culture solution 2a may be done constantly or occasionally depending on a culture state. A supply flow rate of the substrate gas or the predetermined constituent (CO, H₂ or the like) of the substrate gas from the substrate gas source 3 to the culture tank 10 may be monitored and the culture solution 2a may be discharged when the supply flow rate becomes below a predetermined value. Alternatively, the culture solution 2a may be discharged in an amount corresponding to a growth of the gas-utilizing microorganisms 9 in the culture tank 10. Specifically, a concentration of the gas-utilizing microorganisms 9 in the culture tank 10 may be monitored, and the culture solution 2a may be discharged when the concentration of the gas-utilizing microorganisms 9 becomes higher than a predetermined value. The flow rate of the discharged culture solution 2a may be controlled according to the concentration of the gas-utilizing microorganisms 9 (See FIG. 11).

### <Concentrating Step>

The discharged culture solution 2a is introduced to the non-permeation chamber 53 of the biomass concentration part 50G via the discharge passage 22. Liquid constituents of the culture solution 2a passing through the non-permeation chamber 53 can move to the permeation chamber 52 by permeating the filter 51. On the other hand, solid constituents including the biomass composed of living bodies and dead bodies of the gas-utilizing microorganisms 9 or the like in the culture solution 2a are prohibited from penetrating the filter 51. Therefore, the culture solution 2a is separated into a diluted culture solution 2c (permeated solution) in the permeation chamber 52 and a concentrated culture solution 2e (non-permeated solution) in the non-permeation chamber 53. A biomass concentration, and thus a concentration of the gas-utilizing microorganisms 9, of the diluted culture solution 2c is sufficiently lower than that of the discharged culture solution 2a, and thus that of the culture solution 2 in the culture tank 10. Preferably, the diluted culture solution 2c contains almost no biomass. A biomass concentration of the diluted culture solution 2c of the present system 1G (FIG. 10) may be higher than the biomass concentration of the diluted culture solution 2c of the system 1D, 1E, 1F (FIGS. 4 to 9). A biomass concentration, and thus a concentration of the gas-utilizing microorganisms 9, of the concentrated culture solution 2e is higher than that of the discharged culture solution 2a, and thus that of the culture solution 2 in the culture tank 10. In other words, the gas-utilizing microorganisms 9 are diluted (including complete removal) in the diluted culture solution 2c and the gas-utilizing microorganisms 9 are concentrated in the concentrated culture solution 2e.

### <Returning Step>

The diluted culture solution 2c is returned to the culture tank 10 via the diluted solution return passage 41.

### <Replenishing Step>

The culture medium 2S is replenished from the culture medium source 12 to the culture tank 10 in an amount corresponding to a difference between a discharge flow rate U₀ of the discharged culture solution 2a and a return flow rate U₂ of the diluted culture solution 2c. A replenishment flow rate of the culture medium 2S is equal to a flow rate U₄ (=U₀ - U₂) of the concentrated culture solution 2e. Thereby, an amount of the culture solution 2 in the culture tank 10 can be maintained constant.

Here, since the biomass concentration in the diluted culture solution 2c is almost zero, the biomass concentration in the concentrated culture solution 2e is U₀/(=U₀ - U₂) times the biomass concentration of the culture solution 2, 2a. Accordingly, a rate of concentration at the biomass concentration part 50G can be controlled by controlling the flow rate U₀ of the discharged culture solution 2a and the flow rate U₂ of the diluted culture solution 2c.

### <Sending Out Step to Subsequent Steps>

The concentrated culture solution 2e is sent out from the culture apparatus 1x to subsequent steps via the concentrated solution send-out passage 29. Specifically, the concentrated culture solution 2e is introduced to the distillation tower 80 via the concentrated solution send-out passage 29.

### <Extracting Step>

In the distillation tower 80, the concentrated culture solution 2e is distilled and ethanol (valuable material) is extracted. A concentration of ethanol in the culture solution 2 can be as high as possible by making the return flow rate U₂ of the diluted culture solution 2c as high as possible and making the supply flow rate of the culture medium 2S as low as possible. Thus, efficiency of extraction of ethanol at the distillation tower 80 can be enhanced. The extracted ethanol is sent out to the extracted liquid passage 82 from the upper end portion of the distillation tower 80 and provided for various uses via a refining step or the like.

### <Discharge Solution Treating Step>

Extraction residual liquid 2d deposits on the bottom portion of the distillation tower 80. The extraction residual liquid 2d contains a biomass including dead bodies of the gas-utilizing microorganisms 9 in high concentration. The extraction residual liquid 2d is sent out to the discharge solution treatment part 8 from a lower end portion of the distillation tower 80 via the discharge passage 83. The extraction residual liquid 2d is treated anaerobically or aerobically in the discharge solution treatment part 8, thereby the biomass is degraded. Alternatively, the biomass may be aggregated and utilized as a fuel (heat source) for extracting valuable materials.

In the valuable materials generating system 1G, the gas-utilizing microorganisms 9 contained in the discharged culture solution 2a can be condensed and discharged from the culture apparatus 1x. In other words, a portion of the liquid constituents of the discharged culture solution 2a can be separated from the gas-utilizing microorganisms 9 and returned to the culture tank 10 as the diluted culture solution 2c. Accordingly, the discharge flow rate of the culture solution 2 to the out of the system can be decreased, and thereby, waste of the culture solution 2 can be reduced. Moreover, decreasing the replenishment flow rate of the culture medium 2S allows for reduction of cost. It also allows for maintaining an environment inside the culture tank 10 (composition of the culture solution 2 and so on) as constant as possible, thereby, the gas-utilizing microorganisms 9 can be more stably cultured.

The composition of the liquid constituents of the culture solution 2 in the culture tank 10 is not necessarily the same as a composition of the culture medium 2S. Life activities of the gas-utilizing microorganisms 9 may consume or produce some constituents. If a supplied amount of the culture medium 2S were excessively great, the gas-utilizing microorganisms 9 might die of shock due to a sudden change in the environment.

When the supply flow rate of the substrate gas in the substrate gas source 3 is decreased and when the gas-utilizing microorganisms 9 excessively grow to be highly concentrated in the culture tank 10, the liquid sending pump 23 is powered up to increase the flow rate of the discharged culture solution 2a. Thereby, the population of the gas-utilizing microorganisms 9 in the culture tank 10 is reduced, and thereby, an amount of the substrate gas that each of the microorganisms intakes can be secured and an amount of nutrient contents that each of the microorganisms intakes can be secured. Therefore, weakening and death of generally all of the gas-utilizing microorganisms 9 can be avoided. As a result, the gas-utilizing microorganisms 9 can be further stably cultured. Even when the flow rate of the discharged culture solution 2a is increased, waste of the culture solution 2 can be surely constrained by returning the diluted culture solution 2c separated from the discharged culture solution 2a to the culture tank 10 as mentioned above.

### <Eighth Embodiment>

FIG. 11 shows an eighth embodiment of the present invention.

The valuable material generating system 1H further includes a controller 60 (separation ratio control part) and a biomass concentration measuring instrument 61 (microbial concentration measuring instrument). A liquid return pump 42 is disposed in a diluted solution return passage 41.

The biomass concentration measuring instrument 61 is disposed in a discharge passage 22 to measure a biomass concentration of a discharged culture solution 2a. Thus, a concentration of gas-utilizing microorganisms 9 in a culture solution 2 in a culture tank 10 is measured. The biomass concentration measuring instrument 61 may be an optical densitometer that sheds light on the culture solution and measures biomass concentration from an absorptance of the light. By using the optical densitometer, the biomass concentration can be measured in real time.

Alternatively, the biomass concentration measuring instrument 61 may be disposed inside the culture tank 10 and the concentration of the gas-utilizing microorganisms 9 in the culture tank 10 may be directly measured.

Information on the concentration measured by the biomass concentration measuring instrument 61 may be sent to the controller 60. Based on the information on measured concentration, the controller 60 controls an output of the liquid return pump 42, that is a flow rate of a diluted culture solution 2c so that the concentration of the gas-utilizing microorganisms 9 in the culture solution 2 may be a predetermined value. Thereby, a separation ratio between the diluted culture solution 2c and a concentrated culture solution 2e in a biomass concentration portion 50G is controlled. Moreover, the controller 60 achieves a balance between a flow rate of the discharged culture solution 2a and a total flow rate of the return flow rate of the diluted culture solution 2c and a supply flow rate of a culture medium 2S by controlling an output of a liquid sending pump 23. Thereby, an amount of the culture solution 2 in the culture tank 10 can be maintained at a predetermined amount.

Specifically, when the measured biomass concentration is higher than a predetermined value, the liquid return pump 42 is powered up. This causes an amount of liquid permeating a filter 51 at the biomass concentration portion 50G to be increased, thereby causing the return flow rate of the diluted culture solution 2c not containing the gas-utilizing microorganisms 9 to the culture tank 10 to be increased. Since this causes an amount of liquid inside the culture tank 10 to be increased, the liquid sending pump 23 is powered up to increase the flow rate of the discharged culture solution 2a. Therefore, an amount of the gas-utilizing microorganisms 9 removed from the culture tank 10 is increased. Therefore, the concentration of the gas-utilizing microorganisms 9 in the culture tank 10 can be lowered. Conversely, when the measured biomass concentration is lower than the predetermined value, the liquid return pump 42 is powered down. This causes an amount of liquid permeating the filter 51 at the biomass concentration portion 50G to be reduced, thereby causing the return flow rate of the diluted culture solution 2c to the culture tank 10 to be decreased. At the same time, the liquid sending pump 23 is powered down to decrease the discharge flow rate of the culture solution 2a. Therefore, an amount of the gas-utilizing microorganisms 9 removed from the culture tank 10 is reduced. Therefore, the concentration of the gas-utilizing microorganisms 9 in the culture tank 10 can be raised. As a result, the concentration of the gas-utilizing microorganisms 9 in the culture tank 10 can be maintained at a constant level.

As with the valuable materials generating system 1G (FIG. 10), etc., when a supply flow rate of a substrate gas (CO, H₂) from a gas supply passage 31 to the culture tank 10 is below a predetermined value, the population of the gas-utilizing microorganisms 9 in the culture tank 10 is reduced by powering up the liquid sending pump 23 to increase the flow rate of the discharged culture solution 2a. Thereby, death of the entire population of the gas-utilizing microorganisms 9 can be avoided and the gas-utilizing microorganisms 9 can be stably cultured.

The present invention is not limited to the embodiments described above. Various modifications can be made without departing from the scope and spirit of this invention.

For example, in the discharge amount controlling step in the first embodiment (FIG. 1), etc., the discharge amount of the culture solution 2a may be controlled based on the supply flow rate of the entire substrate gas instead of the supply flow rate of the predetermined constituent of the substrate gas. That is, when the supply flow rate of the entire substrate gas is above a predetermined value, the normal operation may be adopted and when the supply flow rate of the substrate gas is below the predetermined value, the discharge amount of the culture solution 2a may be increased. In this case, only the flow meter 32 may be disposed in the gas supply passage 31 and the gas sensor 33 may be omitted. Alternatively, the discharge amount of the culture solution 2a may be controlled based on a temperature or a pressure of the substrate gas or the partial pressure of the predetermined constituent or the like that may reflect the supply situation of the substrate gas or the predetermined constituent.

In the first embodiment (FIG. 1), etc., the storage tank 84 for extraction may be omitted. The discharge passage 22 may continue to the distillation tower 80 not through the storage tank 84 for extraction. Moreover, the discharge passage 22 may continue to the discharge solution treatment part 8 not through the storage tank 84 and the distillation tower 80. In the second embodiment (FIG. 2), the discharge passage 22 may continue to the inlet port of the non-permeation chamber 53 of the biomass concentration portion 50 not through the storage tank 84 and the outlet port of the non-permeation chamber 53 may continue to the distillation tower 80 not through the storage tank 84.

A solid-liquid separator such as a filter and a centrifugal separator may be disposed in the send-out passage 81, 29 to prevent solid constituents from entering in the distillation tower 80. Thereby, a maintenance frequency of the distillation tower 80 can be reduced.

In the seventh embodiment (FIG. 10), etc., during the normal operation, as in the Patent Document 1, etc., the concentrated culture solution 2e after the microorganisms 9 are concentrated and separated may be returned to the culture tank 10 and the diluted culture solution 2c may be sent out to the distillation tower 80 of the subsequent apparatus 1y for extraction of the valuable materials. When a living environment deteriorates such as when the supply flow rate of the substrate gas declines or when the gas-utilizing microorganisms 9 in the culture tank 10 excessively grow, as in the systems 1G, 1H, the diluted culture solution 2c after the microorganisms 9 are concentrated and separated may be returned to the culture tank 10 and the concentrated culture solution 2e may be sent out to the subsequent apparatus 1y.

The distillation tower 80 or the discharge solution treatment part 8 of the subsequent apparatus 1y may be omitted.

It is acceptable as far as the concentration of microorganisms in the diluted culture solution 2c (microorganisms diluted/removed solution) is lower than that of the concentrated culture solution 2e. It is acceptable that the diluted culture solution 2c may contain microorganisms in a concentration that is lower than that of the non-permeated solution 2.

Multiple embodiments may be combined with each other. For example, as with the eighth embodiment (FIG. 11), a controller 60 and a biomass concentration measuring instrument 61 may be added to the first to the seventh embodiments (FIGS. 1 to 10).

### Industrial Applicability

The present invention may be applied to an ethanol generation system for synthesizing ethanol from carbon monoxide generated in an incineration treatment of industrial wastes, for example.

### Explanation of Reference Numerals

- 1x: culture apparatus
- 1y: subsequent apparatus
- 2: culture solution
- 2a: discharged culture solution
- 31: gas supply passage
- 8: discharge solution treatment part
- 9: gas-utilizing microorganisms
- 10: culture tank
- 21: discharge control part
- 2c: diluted culture solution
- 2e: concentrated culture solution
- 28, 29: concentrated solution send-out passage
- 40: diluted solution storage tank
- 41: diluted solution return passage
- 43: rapid replenishment passage
- 45: backwash passage
- 46: cooler (liquid temperature conditioner)
- 47: heat exchanger
- 50, 50G: separation part
- 51: filter (separation film)
- 52: permeation chamber
- 53: non-permeation chamber
- 54: storage tank for concentration
- 58: circulation passage
- 59: filter unit
- 60: controller (separation ratio control part)
- 61: biomass concentration measuring instrument (microbial concentration measuring instrument)
- 80: distillation tower (extraction part)
- 84: storage tank for extraction

## Claims

1. A culture method for culturing gas-utilizing microorganisms (9) that produce valuable materials from a substrate gas by fermentation, the method comprising steps of:
introducing a culture medium (2S) from a culture medium source (12) to the culture tank (10) and culturing the gas-utilizing microorganisms (9) in a culture solution (2) in a culture tank (10);
supplying the substrate gas from a substrate gas source (3) to the culture tank (10); and
controlling a discharge amount of a portion (2a) of the culture solution (2) containing the gas-utilizing microorganisms (9) to be discharged as a discharged culture solution (2a) from the culture tank (10);
**characterized in that** in the step of controlling the discharge amount,
the culture solution (2) is discharged from the culture tank (10) more rapidly than that in a normal mode, in which mode a supply flow rate of the substrate gas or a predetermined constituent thereof is greater than a predetermined value, the culture solution being discharged before a time point when it is known that the supply flow rate will be decreased at the time point in the future due to condition of the substrate gas source (3), or the culture solution being discharged when the supply flow rate of the substrate gas or the predetermined constituent to the culture tank (10) has become equal to or lower than the predetermined value.

2. The culture method according to claim 1, wherein the culture solution (2) is replenished to the culture tank (10) according to an amount of the culture solution (2) rapidly discharged from the culture tank (10).

3. The culture method according to claim 1 or 2, wherein the method further comprises steps of:
separating the discharged culture solution (2a) into a concentrated culture solution (2e) and a diluted culture solution (2c), the gas-utilizing microorganisms (9) being concentrated in the concentrated culture solution (2e), the gas-utilizing microorganisms (9) being diluted in the diluted culture solution (2c);
returning the diluted culture solution (2c) to the culture tank (10); and
sending out the concentrated culture solution (2e) to subsequent apparatus (1y) including an extraction part (80) that extracts the valuable materials or a storage tank (84) for extraction or a discharge solution treatment part (8).

4. The culture method according to claim 3, wherein in the step of separating, the discharged culture solution (2a) is circulated along a circulation passage (58) in which, of a filter (51) and a storage tank (54) for concentration, at least the filter (51) is disposed.

5. The culture method according to claim 3 or 4, wherein the method further comprises steps of: monitoring a concentration of the gas-utilizing microorganisms (9) in the culture solution (2) in the culture tank (10) or in the discharged culture solution (2a); and
controlling a separation ratio between the concentrated culture solution (2e) and the diluted culture solution (2c) so that the concentration may be a predetermined concentration.

6. The culture method according to claim 1 or 2, wherein the method further comprises steps of:
separating the discharged culture solution (2a) into a concentrated culture solution (2e) and a diluted culture solution (2c), the gas-utilizing microorganisms (9) being concentrated in the concentrated culture solution (2e), the gas-utilizing microorganisms (9) being diluted in the diluted culture solution (2c);
storing the diluted culture solution (2c); and
returning the stored diluted culture solution (2c) to the culture tank (10) at the same time as or slightly before or after the rapid discharge of the culture solution (2).

7. The culture method according to claim 6, further comprising:
returning the diluted culture solution (2c) obtained in the step of separating to the culture tank (10) after the rapid discharge until the supply flow rate is recovered to the predetermined value or higher; and
sending out the concentrated culture solution (2e) obtained in the step of separating to the subsequent apparatus (1y) including the extraction part (80) that extracts the valuable materials or a storage tank (84) for extraction or the discharge solution treatment part (8).

8. The culture method according to claim 6 or 7, further comprising running in a state recovery operation mode after the troubles at the substrate gas source (3A) is resolved and the supply flow rate of the substrate gas becomes returnable to the predetermined value or higher, wherein the concentrated culture solution (2e) obtained in the step of separating is returned to the culture tank (10) in the state recovery operation mode.

9. The culture method according to any one of the claims 6 to 8, further comprising a step of making a temperature of the diluted culture solution (2c) under storage higher or lower than a temperature of the culture tank (10).

10. The culture method according to claim 9, further comprising a step of exchanging heat between the diluted culture solution (2c) to be returned to the culture tank (10) and the rapidly discharged culture solution (2a).

11. The culture method according to any one of the claims 6 to 10, further comprising a step of backwashing the filter (51) for the step of separating by at least a portion of the diluted culture solution (2c) to be returned to the culture tank (10).

12. A culture apparatus (1x) for culturing gas-utilizing microorganisms (9) that produce valuable materials from a substrate gas by fermentation, the apparatus (1x) comprising:
a culture tank (10) that receives a culture solution (2), the gas-utilizing microorganisms (9) being cultured in the culture solution (2);
a culture medium source (12) connected to the culture tank (10), a culture medium (2S) of the culture solution (2) being stored in the culture medium source (12);
a gas supply passage (31) connected to the culture tank (10), the substrate gas from a substrate gas source (3) being supplied to the culture solution (2) in the culture tank (10) through the gas supply passage (31); and
a discharge control part (21) that includes a liquid sending pump (23) and that controls a discharge amount of a portion (2a) of the culture solution (2) containing the gas-utilizing microorganisms (9) in the culture tank (10) to be discharged as a discharged culture solution (2a); **characterized in that**
the apparatus (1x) further comprises a gas flow meter (32) and a gas sensor (33) disposed in intermediate portions of the gas supply passage (31),
the culture solution (2) is discharged more rapidly than that in a normal mode, in which mode a supply flow rate of the substrate gas or a predetermined constituent thereof is greater than a predetermined value, by the discharge control part (21) including the liquid sending pump (23) from the culture tank (10) when it is known that the supply flow rate will be decreased at the time point in the future due to condition of the substrate gas source (3), or the culture solution being discharged when the supply flow rate based on results of measurements with the flow meter (31) and with the gas sensor (33) has become equal to or lower than the predetermined value,
wherein the apparatus (1x) further comprises:
a separation part (50; 50G; 59) that separates the discharged culture solution (2a) into a concentrated culture solution (2e) and a diluted culture solution (2c), the gas-utilizing microorganisms (9) being concentrated in the concentrated culture solution (2e), the gas-utilizing microorganisms (9) being diluted in the diluted culture solution (2c);
a diluted solution return passage (41) that returns the diluted culture solution (2c) to the culture tank (10); and
a concentrated solution send-out passage (28; 29) that sends out the concentrated culture solution (2e) to subsequent apparatus (1y) including an extraction part (80) that extracts the valuable materials or a storage tank (84) for extraction or a discharge solution treatment part (8).

13. The culture apparatus (1x) according to claim 12, wherein the apparatus (1x) further comprises a rapid replenishment passage (43), the culture solution (2) being replenished to the culture tank (10) according to an amount of the culture solution (2) rapidly discharged through the rapid replenishment passage (43).

14. The culture apparatus (1x) according to claim 12 or 13, wherein the separation part (50) comprises: a circulation passage (58), the concentrated culture solution (2e) being circulated in the circulation passage (58); and a filter (51) disposed in the circulation passage (58).

15. The culture apparatus (1x) according to claim 14, wherein a storage tank (54) for concentration is disposed in the circulation passage (58).

16. The culture apparatus (1x) according to any one of the claims 12 to 15, wherein the apparatus (1x) further comprises:
a microbial concentration measuring instrument (61) that measures a concentration of the gas-utilizing microorganisms (9) in the culture solution (2) in the culture tank (10); and
a separation ratio control part (60) that controls a separation ratio between the concentrated culture solution (2e) and the diluted culture solution (2c) in the separation part (50G) so that the concentration may be a predetermined concentration.

17. The culture apparatus (1x) according to claim 13, wherein: the apparatus (1x) further comprises:
a separation part (59) that separates the discharged culture solution (2a) into a concentrated culture solution (2e) and a diluted culture solution (2c), the gas-utilizing microorganisms (9) being concentrated in the concentrated culture solution (2e), the gas-utilizing microorganisms (9) being diluted in the diluted culture solution (2c); and
a diluted solution storage tank (40) that stores the diluted culture solution (2c); and wherein the rapid replenishment passage (43) extends from the diluted solution storage tank (40) to the culture tank (10).

18. The culture apparatus (1x) according to claim 17, wherein the diluted solution storage tank (40) is provided with a liquid temperature conditioner (46) that makes a temperature of the diluted culture solution (2c) higher or lower than a temperature of the culture tank (10).

19. The culture apparatus (1x) according to claim 18, further comprising a heat exchanger (47) that exchanges heat between the diluted culture solution (2c) in the rapid replenishment passage (43) and the rapidly discharged culture solution (2a).

## Patentansprüche

1. Verfahren zur Kultivierung von gasverwertenden Mikroorganismen (9), die durch Fermentation aus einem Substratgas wertvolle Stoffe erzeugen, wobei das Verfahren folgende Schritte umfasst:
Einleiten eines Kulturmediums (2S) aus einer Kulturmediumquelle (12) in den Kulturtank (10) und Kultivieren der gasverwertenden Mikroorganismen (9) in einer Kulturlösung (2) in einem Kulturtank (10);
Zuführen des Substratgases aus einer Substratgasquelle (3) in den Kulturtank (10); und
Steuern einer Abgabemenge eines Teils (2a) der Kulturlösung (2), der die gasverwertenden Mikroorganismen (9) enthält, die als abgelassene Kulturlösung (2a) aus dem Kulturtank (10) abgelassen werden soll;
**dadurch gekennzeichnet, dass** im Schritt des Steuerns der Abgabemenge die Kulturlösung (2) schneller aus dem Kulturtank (10) abgelassen wird als in einem Normalmodus, in dem die Zufuhrrate des Substratgases oder eines vorbestimmten Bestandteils davon größer als ein vorbestimmter Wert ist, wobei die Kulturlösung vor einem Zeitpunkt abgelassen wird, zu dem bekannt ist, dass die Zufuhrrate zu diesem Zeitpunkt in der Zukunft aufgrund des Zustands der Substratgasquelle (3) verringert wird, oder die Kulturlösung abgelassen wird, wenn die Zufuhrrate des Substratgases oder des vorbestimmten Bestandteils zum Kulturtank (10) gleich oder kleiner als der vorbestimmte Wert geworden ist.

2. Kulturverfahren nach Anspruch 1, wobei die Kulturlösung (2) entsprechend der Menge der aus dem Kulturtank (10) schnell abgelassenen Kulturlösung (2) in den Kulturtank (10) nachgeführt wird.

3. Kulturverfahren nach Anspruch 1 oder 2, wobei das Verfahren ferner die folgenden Schritte umfasst:
Trennen der abgelassenen Kulturlösung (2a) in eine konzentrierte Kulturlösung (2e) und eine verdünnte Kulturlösung (2c), wobei die gasverwertenden Mikroorganismen (9) in der konzentrierten Kulturlösung (2e) konzentriert sind und wobei die gasverwertenden Mikroorganismen (9) in der verdünnten Kulturlösung (2c) verdünnt sind;
Zurückführen der verdünnten Kulturlösung (2c) in den Kulturtank (10); und
Weiterleiten der konzentrierten Kulturlösung (2e) zu einer nachfolgenden Vorrichtung (1y), die einen Extraktionsabschnitt (80) zur Extraktion der wertvollen Stoffe oder einen Speichertank (84) zur Extraktion oder einen Abschnitt (8) zur Behandlung der Abflusslösung umfasst.

4. Kulturverfahren nach Anspruch 3, wobei im Trennschritt die abgelassene Kulturlösung (2a) entlang eines Zirkulationskanals (58) zirkuliert wird, in dem von einem Filter (51) und einem Speichertank (54) zur Konzentration mindestens der Filter (51) angeordnet ist.

5. Kulturverfahren nach Anspruch 3 oder 4, wobei das Verfahren ferner die folgenden Schritte umfasst: Überwachen einer Konzentration der gasverwertenden Mikroorganismen (9) in der Kulturlösung (2) im Kulturtank (10) oder in der abgelassenen Kulturlösung (2a); und
Steuern eines Trennverhältnisses zwischen der konzentrierten Kulturlösung (2e) und der verdünnten Kulturlösung (2c), so dass die Konzentration eine vorbestimmte Konzentration sein kann.

6. Kulturverfahren nach Anspruch 1 oder 2, wobei das Verfahren ferner die folgenden Schritte umfasst:
Trennen der abgelassenen Kulturlösung (2a) in eine konzentrierte Kulturlösung (2e) und eine verdünnte Kulturlösung (2c), wobei die gasverwertenden Mikroorganismen (9) in der konzentrierten Kulturlösung (2e) konzentriert sind und wobei die gasverwertenden Mikroorganismen (9) in der verdünnten Kulturlösung (2c) verdünnt sind;
Lagern der verdünnten Kulturlösung (2c); und
Rückführen der gelagerten verdünnten Kulturlösung (2c) in den Kulturtank (10) gleichzeitig mit oder kurz vor oder nach dem schnellen Ablassen der Kulturlösung (2).

7. Kulturverfahren nach Anspruch 6, ferner umfassend:
Zurückführen der im Trennschritt erhaltenen verdünnten Kulturlösung (2c) in den Kulturtank (10) nach dem schnellen Ablassen, bis die Zufuhrrate wieder auf den vorgegebenen Wert oder höher angestiegen ist; und
Weiterleiten der im Trennschritt erhaltenen konzentrierten Kulturlösung (2e) an die nachfolgende Vorrichtung (1y), die den Extraktionsabschnitt (80) zur Extraktion der wertvollen Stoffe oder einen Speichertank (84) zur Extraktion oder den Abschnitt (8) zur Behandlung der Abflusslösung umfasst.

8. Kulturverfahren nach Anspruch 6 oder 7, ferner umfassend: den Betrieb in einem Betriebsmodus zur Wiederherstellung des Zustands, nachdem die Störungen an der Substratgasquelle (3A) behoben sind und die Zufuhrrate des Substratgases wieder auf den vorbestimmten Wert oder höher zurückkehren kann, wobei die im Trennschritt erhaltene konzentrierte Kulturlösung (2e) im Betriebsmodus zur Wiederherstellung des Zustands in den Kulturtank (10) zurückgeführt wird.

9. Kulturverfahren nach einem der Ansprüche 6 bis 8, ferner umfassend einen Schritt, bei dem die Temperatur der verdünnten Kulturlösung (2c) während der Lagerung höher oder niedriger als die Temperatur des Kulturtanks (10) eingestellt wird.

10. Kulturverfahren nach Anspruch 9, ferner umfassend einen Schritt, bei dem Wärme zwischen der verdünnten Kulturlösung (2c), die in den Kulturtank (10) zurückgeführt werden soll, und der schnell abgelassenen Kulturlösung (2a) ausgetauscht wird.

11. Kulturverfahren nach einem der Ansprüche 6 bis 10, ferner umfassend einen Schritt, bei dem der Filter (51) für den Trennschritt mit mindestens einem Teil der verdünnten Kulturlösung (2c), die in den Kulturtank (10) zurückgeführt werden soll, rückgespült wird.

12. Kulturvorrichtung (1x) zur Kultivierung gasverwertender Mikroorganismen (9), die durch Fermentation aus einem Substratgas wertvolle Stoffe produzieren, wobei die Vorrichtung (1x) umfasst:
einen Kulturtank (10), der eine Kulturlösung (2) aufnimmt, wobei die gasverwertenden Mikroorganismen (9) in der Kulturlösung (2) kultiviert werden;
eine mit dem Kulturtank (10) verbundene Kulturmediumquelle (12), wobei ein Kulturmedium (2S) der Kulturlösung (2) in der Kulturmediumquelle (12) gespeichert ist;
einen mit dem Kulturtank (10) verbundenen Gaszufuhrkanal (31), wobei das Substratgas aus einer Substratgasquelle (3) über den Gaszufuhrkanal (31) der Kulturlösung (2) im Kulturtank (10) zugeführt wird; und
einen Auslasssteuerungsabschnitt (21), der eine Flüssigkeitsförderpumpe (23) umfasst und eine Auslassmenge eines Teils (2a) der Kulturlösung (2), die die gasverwertenden Mikroorganismen (9) im Kulturtank (10) enthält, steuert, der als Auslasskulturlösung (2a) ausgegeben werden soll; **dadurch gekennzeichnet, dass**
die Vorrichtung (1x) ferner einen Gasdurchflussmesser (32) und einen Gassensor (33) umfasst, die in Zwischenabschnitten des Gaszufuhrkanals (31) angeordnet sind,
die Kulturlösung (2) durch den Auslasssteuerungsabschnitt (21), der die Flüssigkeitsförderpumpe (23) umfasst, schneller aus dem Kulturtank (10) abgelassen wird als in einem Normalmodus, wobei in diesem Modus die Zufuhrrate des Substratgases oder eines vorbestimmten Bestandteils davon größer als ein vorbestimmter Wert ist, wenn bekannt ist, dass die Zufuhrrate zu einem Zeitpunkt in der Zukunft aufgrund des Zustands der Substratgasquelle (3) sinken wird, oder die Kulturlösung abgelassen wird, wenn die Zufuhrrate, basierend auf den Messergebnissen des Durchflussmessers (31) und des Gassensors (33), gleich oder kleiner als der vorbestimmte Wert geworden ist,
wobei die Vorrichtung (1x) ferner umfasst:
einen Separationsteil (50; 50G; 59), der die abgelassene Kulturlösung (2a) in eine konzentrierte Kulturlösung (2e) und eine verdünnte Kulturlösung (2c) trennt, wobei die gasverwertenden Mikroorganismen (9) in der konzentrierten Kulturlösung (2e) konzentriert sind, wobei die gasverwertenden Mikroorganismen (9) in der verdünnten Kulturlösung (2c) verdünnt sind;
einen Rückführkanal (41) für die verdünnte Lösung, der die verdünnte Kulturlösung (2c) in den Kulturtank (10) zurückführt; und
einen Auslasskanal (28; 29) für die konzentrierte Lösung, der die konzentrierte Kulturlösung (2e) zu einer nachfolgenden Vorrichtung (1y) weiterleitet, die einen Extraktionsabschnitt (80) zur Extraktion der wertvollen Stoffe oder einen Speichertank (84) zur Extraktion oder einen Behandlungsabschnitt (8) für die Abflusslösung umfasst.

13. Kulturvorrichtung (1x) nach Anspruch 12, wobei die Vorrichtung (1x) ferner einen Schnellnachfüllkanal (43) umfasst, wobei die Kulturlösung (2) entsprechend einer Menge der Kulturlösung (2), die schnell durch den Schnellnachfüllkanal (43) abgelassen wird, in den Kulturtank (10) nachgefüllt wird.

14. Kulturvorrichtung (1x) nach Anspruch 12 oder 13, wobei der Separationsteil (50) umfasst: einen Zirkulationskanal (58), wobei die konzentrierte Kulturlösung (2e) in dem Zirkulationskanal (58) zirkuliert wird; und einen Filter (51), der in dem Zirkulationskanal (58) angeordnet ist.

15. Kulturvorrichtung (1x) nach Anspruch 14, wobei ein Speichertank (54) zur Konzentration in dem Zirkulationskanal (58) angeordnet ist.

16. Kulturvorrichtung (1x) nach einem der Ansprüche 12 bis 15, wobei die Vorrichtung (1x) ferner umfasst:
ein Messgerät zur Bestimmung der mikrobiellen Konzentration (61), das die Konzentration der gasverwertenden Mikroorganismen (9) in der Kulturlösung (2) im Kulturtank (10) misst; und
einen Teil zur Regelung des Trennverhältnisses (60), der ein Trennverhältnis zwischen der konzentrierten Kulturlösung (2e) und der verdünnten Kulturlösung (2c) im Separationsteil (50G) so steuert, dass die Konzentration eine vorbestimmte Konzentration sein kann.

17. Kulturvorrichtung (1x) nach Anspruch 13, wobei die Vorrichtung (1x) ferner umfasst:
einen Separationsteil (59), der die abgelassene Kulturlösung (2a) in eine konzentrierte Kulturlösung (2e) und eine verdünnte Kulturlösung (2c) trennt, wobei die gasverwertenden Mikroorganismen (9) in der konzentrierten Kulturlösung (2e) konzentriert sind, wobei die gasverwertenden Mikroorganismen (9) in der verdünnten Kulturlösung (2c) verdünnt sind; und
einen Vorratsbehälter für verdünnte Lösung (40), der die verdünnte Kulturlösung (2c) speichert; und wobei sich der Schnellnachfüllkanal (43) vom Vorratsbehälter für verdünnte Lösung (40) zum Kulturtank (10) erstreckt.

18. Kulturvorrichtung (1x) nach Anspruch 17, wobei der Vorratsbehälter für verdünnte Lösung (40) mit einem Flüssigkeitstemperaturregler (46) versehen ist, der die Temperatur der verdünnten Kulturlösung (2c) höher oder niedriger als die Temperatur des Kulturbehälters (10) macht.

19. Kulturvorrichtung (1x) nach Anspruch 18, ferner umfassend einen Wärmetauscher (47), der Wärme zwischen der verdünnten Kulturlösung (2c) im Schnellnachfüllkanal (43) und der schnell abgelassenen Kulturlösung (2a) austauscht.

## Revendications

1. Procédé de culture pour mettre en culture des micro-organismes utilisant du gaz (9) qui produisent des matières valorisables à partir d'un gaz support par fermentation, le procédé comportant les étapes consistant à :
introduire un milieu de culture (2S) provenant d'une source de milieu de culture (12) dans un réservoir de culture (10) et mettre en culture les micro-organismes utilisant du gaz (9) dans une solution de culture (2) dans le réservoir de culture (10) ;
fournir le gaz support provenant d'une source de gaz support (3) au réservoir de culture (10) ; et
commander une quantité d'évacuation d'une portion (2a) de la solution de culture (2) contenant les micro-organismes utilisant du gaz (9) à évacuer sous forme de solution de culture (2a) évacuée du réservoir de culture (10) ;
**caractérisé en ce qu'**à l'étape de commande de la quantité d'évacuation,
la solution de culture (2) est évacuée du réservoir de culture (10) plus rapidement que dans un mode normal, mode dans lequel un débit d'alimentation du gaz support ou d'un constituant prédéterminé de celui-ci est supérieur à une valeur prédéterminée, la solution de culture étant évacuée avant un instant où l'on sait que le débit d'alimentation diminuera à un instant dans le futur en raison de l'état de la source de gaz support (3), ou la solution de culture étant évacuée lorsque le débit d'alimentation du gaz support ou du constituant prédéterminé vers le réservoir de culture (10) est devenu égal ou inférieur à la valeur prédéterminée.

2. Procédé de culture selon la revendication 1, dans lequel le réservoir de culture (10) est réapprovisionné en solution de culture (2) en fonction d'une quantité de la solution de culture (2) rapidement évacuée du réservoir de culture (10).

3. Procédé de culture selon la revendication 1 ou 2, dans lequel le procédé comporte en outre les étapes consistant à :
séparer la solution de culture évacuée (2a) en une solution de culture concentrée (2e) et une solution de culture diluée (2c), les micro-organismes utilisant du gaz (9) étant concentrés dans la solution de culture concentrée (2e), les micro-organismes utilisant du gaz (9) étant dilués dans la solution de culture diluée (2c) ;
renvoyer la solution de culture diluée (2c) dans le réservoir de culture (10) ; et
transférer la solution de culture concentrée (2e) dans un appareil suivant (1y) incluant une partie d'extraction (80) qui extrait les matières valorisables, ou un réservoir de stockage (84) pour extraction, ou une partie de traitement de solution d'évacuation (8).

4. Procédé de culture selon la revendication 3, dans lequel, à l'étape de séparation, la solution de culture évacuée (2a) circule le long d'un passage de circulation (58) dans lequel, parmi un filtre (51) et un réservoir de stockage (54) pour concentration, au moins le filtre (51) est disposé.

5. Procédé de culture selon la revendication 3 ou 4, dans lequel le procédé comporte en outre les étapes consistant à : surveiller une concentration des micro-organismes utilisant du gaz (9) dans la solution de culture (2) dans le réservoir de culture (10) ou dans la solution de culture évacuée (2a) ; et
commander un taux de séparation entre la solution de culture concentrée (2e) et la solution de culture diluée (2c) de sorte que la concentration peut être une concentration prédéterminée.

6. Procédé de culture selon la revendication 1 ou 2, dans lequel le procédé comporte en outre les étapes consistant à :
séparer la solution de culture évacuée (2a) en une solution de culture concentrée (2e) et une solution de culture diluée (2c), les micro-organismes utilisant du gaz (9) étant concentrés dans la solution de culture concentrée (2e), les micro-organismes utilisant du gaz (9) étant dilués dans la solution de culture diluée (2c) ;
stocker la solution de culture diluée (2c) ; et
renvoyer la solution de culture diluée (2c) stockée dans le réservoir de culture (10) en même temps ou légèrement avant ou après l'évacuation rapide de la solution de culture (2).

7. Procédé de culture selon la revendication 6, comportant en outre de :
renvoyer la solution de culture diluée (2c) obtenue à l'étape de séparation dans le réservoir de culture (10) après l'évacuation rapide jusqu'à ce que le débit d'alimentation soit revenu à la valeur prédéterminée ou à une valeur plus élevée ; et
transférer la solution de culture concentrée (2e) obtenue à l'étape de séparation dans l'appareil suivant (1y) incluant la partie d'extraction (80) qui extrait les matières valorisables, ou un réservoir de stockage (84) pour extraction, ou la partie de traitement de solution d'évacuation (8).

8. Procédé de culture selon la revendication 6 ou 7, comportant en outre l'exécution d'un mode de fonctionnement avec rétablissement d'état une fois que les problèmes au niveau de la source de gaz support (3A) ont été résolus et que le débit d'alimentation du gaz support peut être ramené à la valeur prédéterminée ou à une valeur plus élevée, dans lequel la solution de culture concentrée (2e) obtenue à l'étape de séparation est renvoyée dans le réservoir de culture (10) dans le mode de fonctionnement avec rétablissement d'état.

9. Procédé de culture selon l'une quelconque des revendications 6 à 8, comportant en outre une étape consistant à augmenter ou réduire une température de la solution de culture diluée (2c) en cours de stockage par rapport à une température du réservoir de culture (10).

10. Procédé de culture selon la revendication 9, comportant en outre une étape consistant à échanger de la chaleur entre la solution de culture diluée (2c) à renvoyer dans le réservoir de culture (10) et la solution de culture (2a) rapidement évacuée.

11. Procédé de culture selon l'une quelconque des revendications 6 à 10, comportant en outre une étape de lavage à contre-courant du filtre (51) pendant l'étape de séparation par au moins une portion de la solution de culture diluée (2c) à renvoyer dans le réservoir de culture (10).

12. Appareil de culture (1x) pour mettre en culture des micro-organismes utilisant du gaz (9) qui produisent des matières valorisables à partir d'un gaz support par fermentation, l'appareil (1x) comportant :
un réservoir de culture (10) qui reçoit une solution de culture (2), les micro-organismes utilisant du gaz (9) étant mis en culture dans la solution de culture (2) ;
une source de milieu de culture (12) reliée au réservoir de culture (10), un milieu de culture (2S) de la solution de culture (2) étant stocké dans la source de milieu de culture (12) ;
un passage d'alimentation en gaz (31) relié au réservoir de culture (10), le gaz support provenant d'une source de gaz support (3) étant fourni à la solution de culture (2) dans le réservoir de culture (10) par le passage d'alimentation en gaz (31) ; et
une partie de commande d'évacuation (21) qui inclut une pompe de transfert de liquide (23) et qui commande une quantité d'évacuation d'une portion (2a) de la solution de culture (2) contenant les micro-organismes utilisant du gaz (9) dans le réservoir de culture (10) à évacuer sous forme de solution de culture évacuée (2a) ; **caractérisé en ce que**
l'appareil (1x) comporte en outre un débitmètre de gaz (32) et un capteur de gaz (33) disposés dans des portions intermédiaires du passage d'alimentation en gaz (31),
la solution de culture (2) est évacuée plus rapidement que dans un mode normal, mode dans lequel un débit d'alimentation du gaz support ou d'un constituant prédéterminé de celui-ci est supérieur à une valeur prédéterminée, par la partie de commande d'évacuation (21) incluant la pompe de transfert de liquide (23) à partir du réservoir de culture (10) lorsqu'on sait que le débit d'alimentation diminuera à un instant dans le futur en raison de l'état de la source de gaz support (3), ou la solution de culture étant évacuée lorsque le débit d'alimentation basé sur des résultats de mesures effectuées avec le débitmètre (31) et avec le capteur de gaz (33) est devenu égal ou inférieur à la valeur prédéterminée,
dans lequel l'appareil (1x) comporte en outre :
une partie de séparation (50 ; 50G ; 59) qui sépare la solution de culture évacuée (2a) en une solution de culture concentrée (2e) et une solution de culture diluée (2c), les micro-organismes utilisant du gaz (9) étant concentrés dans la solution de culture concentrée (2e), les micro-organismes utilisant du gaz (9) étant dilués dans la solution de culture diluée (2c) ;
un passage de retour de solution diluée (41) qui renvoie la solution de culture diluée (2c) dans le réservoir de culture (10) ; et
un passage de transfert de solution concentrée (28 ; 29) qui transfère la solution de culture concentrée (2e) dans un appareil suivant (1y) incluant une partie d'extraction (80) qui extrait les matières valorisables, ou un réservoir de stockage (84) pour extraction, ou une partie de traitement de solution d'évacuation (8).

13. Appareil de culture (1x) selon la revendication 12, dans lequel l'appareil (1x) comporte en outre un passage de réapprovisionnement rapide (43), le réservoir de culture (10) étant réapprovisionné en solution de culture (2) en fonction d'une quantité de la solution de culture (2) rapidement évacuée par le passage de réapprovisionnement rapide (43).

14. Appareil de culture (1x) selon la revendication 12 ou 13, dans lequel la partie de séparation (50) comporte : un passage de circulation (58), la solution de culture concentrée (2e) circulant dans le passage de circulation (58) ; et un filtre (51) étant disposé dans le passage de circulation (58).

15. Appareil de culture (1x) selon la revendication 14, dans lequel un réservoir de stockage (54) pour concentration est disposé dans le passage de circulation (58).

16. Appareil de culture (1x) selon l'une quelconque des revendications 12 à 15, dans lequel l'appareil (1x) comporte en outre :
un instrument de mesure de concentration microbienne (61) qui mesure une concentration des micro-organismes utilisant du gaz (9) dans la solution de culture (2) dans le réservoir de culture (10) ; et
une partie de commande de taux de séparation (60) qui commande un taux de séparation entre la solution de culture concentrée (2e) et la solution de culture diluée (2c) dans la partie de séparation (50G) de sorte que la concentration peut être une concentration prédéterminée.

17. Appareil de culture (1x) selon la revendication 13, dans lequel l'appareil (1x) comporte en outre :
une partie de séparation (59) qui sépare la solution de culture évacuée (2a) en une solution de culture concentrée (2e) et une solution de culture diluée (2c), les micro-organismes utilisant du gaz (9) étant concentrés dans la solution de culture concentrée (2e), les micro-organismes utilisant du gaz (9) étant dilués dans la solution de culture diluée (2c) ; et
un réservoir de stockage de solution diluée (40) qui stocke la solution de culture diluée (2c) ; et dans lequel le passage de réapprovisionnement rapide (43) s'étend du réservoir de stockage de solution diluée (40) au réservoir de culture (10).

18. Appareil de culture (1x) selon la revendication 17, dans lequel le réservoir de stockage de solution diluée (40) est muni d'un élément de conditionnement de température de liquide (46) qui augmente ou réduit une température de la solution de culture diluée (2c) par rapport à une température du réservoir de culture (10).

19. Appareil de culture (1x) selon la revendication 18, comportant en outre un échangeur de chaleur (47) qui échange de la chaleur entre la solution de culture diluée (2c) dans le passage de réapprovisionnement rapide (43) et la solution de culture (2a) rapidement évacuée.
